# EUROPEAN PATENT SPECIFICATION

(11) **EP 3 765 630 B1**
(45) Date of publication and mention of the grant of the patent: **28.08.2024**
(21) Application number: 19767124.1
(22) Date of filing: 14.03.2019
(51) Int. Cl.: C12Q 1/68, G01N 33/50, G01N 33/68

(54) **PRECISION MEDICINE FOR PAIN: DIAGNOSTIC BIOMARKERS, PHARMACOGENOMICS, AND REPURPOSED DRUGS**
PRÄZISIONSARZNEIMITTEL FÜR SCHMERZEN: DIAGNOSTISCHE BIOMARKER, PHARMAKOGENOMIK UND UMGEWIDMETE ARZNEIMITTEL
MÉDICAMENT DE PRÉCISION POUR LA DOULEUR : BIOMARQUEURS DE DIAGNOSTIC, PHARMACOGÉNOMIQUE ET MÉDICAMENTS REPOSITIONNÉS

(30) Priority: 14.03.2018 US 201862642789 P
(43) Date of publication of application: 20.01.2021
(73) Proprietor: INDIANA UNIVERSITY RESEARCH AND TECHNOLOGY CORPORATION, Indianapolis, IN 46202 (US); UNITED STATES GOVERNMENT as represented by THE DEPARTMENT OF VETERANS AFFAIRS, Washington, DC 20420 (US)
(72) Inventor: NICULESCU, Alexander Bogden, Indianapolis, IN 46202 (US)
(74) Representative: V.O.
(86) International application number: PCT/US2019/022305
(87) International publication number: WO 2019/178379

(56) References cited:
- US-A1- 2016 355 584
- A B NICULESCU ET AL: "Precision medicine for suicidality: from universality to subtypes and personalization", MOLECULAR PSYCHIATRY, vol. 22, no. 9, 15 August 2017 (2017-08-15), London, pages 1250 - 1273, XP055732769, ISSN: 1359-4184, DOI: 10.1038/mp.2017.128
- WEBER KATHRYN T. ET AL: "Serum levels of the proinflammatory cytokine interleukin-6 vary based on diagnoses in individuals with lumbar intervertebral disc diseases", ARTHRITIS RESEARCH & THERAPY, vol. 18, no. 1, 1 December 2016 (2016-12-01), XP055894463, Retrieved from the Internet <URL:https://arthritis-research.biomedcentral.com/track/pdf/10.1186/s13075-015-0887-8.pdf> DOI: 10.1186/s13075-015-0887-8
- FORDYCE C B ET AL: "Elevated serum inflammatory markers in post-poliomyelitis syndrome", JOURNAL OF NEUROLOGICAL SCIENCES, ELSEVIER SCIENTIFIC PUBLISHING CO, AMSTERDAM, NL, vol. 271, no. 1-2, 15 August 2008 (2008-08-15), pages 80 - 86, XP022797272, ISSN: 0022-510X, [retrieved on 20080512], DOI: 10.1016/J.JNS.2008.03.015
- JIN EUN-HEUI ET AL: "Genome-Wide Expression Profiling of Complex Regional Pain Syndrome", PLOS ONE, vol. 8, no. 11, 1 January 2013 (2013-01-01), pages e79435, XP055896300, Retrieved from the Internet <URL:https://www.ncbi.nlm.nih.gov/pmc/articles/PMC3828360/pdf/pone.0079435.pdf> DOI: 10.1371/journal.pone.0079435
- LUCHTING BENJAMIN ET AL: "Soluble intercellular adhesion molecule-1: a potential biomarker for pain intensity in chronic pain patients", BIOMARKERS IN MEDICINE, vol. 11, no. 3, 1 March 2017 (2017-03-01), UK, pages 265 - 276, XP093019141, ISSN: 1752-0363, Retrieved from the Internet <URL:https://www.ncbi.nlm.nih.gov/pmc/articles/PMC5705790/pdf/bmm-11-265.pdf> DOI: 10.2217/bmm-2016-0246
- SCHISTAD E I ET AL: "Association between baseline IL-6 and 1-year recovery in lumbar radicular pain", EUROPEAN JOURNAL OF PAIN, SAUNDERS, LONDON, GB, vol. 18, no. 10, 2 April 2014 (2014-04-02), pages 1394 - 1401, XP072002328, ISSN: 1090-3801, DOI: 10.1002/J.1532-2149.2014.502.X
- KRAYCHETE D C ET AL: "Proinflammatory Cytokines in Patients with Neuropathic Pain Treated with Tramadol", REVISTA BRASILEIRA DE ANESTESIOLOGIA, RIO DE JANEIRO, BR, vol. 59, no. 3, 1 May 2009 (2009-05-01), pages 297 - 303, XP027246099, ISSN: 0034-7094, [retrieved on 20090501]
- BYSTROM ET AL.: "Response to Treatment with TNFα Inhibitors in Rheumatoid Arthritis Is Associated with High Levels of GM-CSF and GM-CSF+T Lymphocytes", CLINICAL REVIEWS IN ALLERGY AND IMMUNOLOGY, vol. 53, no. 2, 2017, pages 265 - 276, XP036319125
- KONDRATIEVA ET AL.: "Biomarkers of migraine: Part 1 - Genetic markers", JOURNAL OF NEUROLOGICAL SCIENCES, vol. 369, 2016, pages 63 - 76, XP029735312, DOI: 10.1016/j.jns.2016.08.008
- SOVEYD ET AL.: "Molecular mechanisms of omega-3 fatty acids in the migraine headache", IRANIAN JOURNAL OF NEUROLOGY, vol. 16, no. 4, 2017, pages 210 - 217, XP055642483
- OHTA ET AL.: "Clinical significance of the reduced expression of G protein gamma 7 (GNG7) in oesophageal cancer", BRITISH JOURNAL OF CANCER, vol. 98, no. 2, 2008, pages 410 - 417, XP055642487
- SCHWINDINGER ET AL.: "Synergistic roles for G-protein [gamma]3 and [gamma]7 subtypes in seizure susceptibility as revealed in double knock-out mice", JOURNAL OF BIOLOGICAL CHEMISTRY, vol. 287, no. 10, 2012, pages 7121 - 7133, XP055642491

## Description

### STATEMENT OF GOVERNMENT SUPPORT

This invention was made with government support under OD007363 awarded by the National Institutes of Health and CX000139 merit award by the Veterans Administration. The government has certain rights in the invention.

### BACKGROUND OF THE DISCLOSURE

The present disclosure relates generally to methods for objectively determining and predicting pain. More particularly, the present disclosure relates to methods for tracking pain intensity, predicting levels of pain and predicting future medical facility visits for pain. Also disclosed are drugs and natural compounds identified as candidates for treating pain using biomarker gene expression signatures. Niculescu et al. (Molecular Psychiatry, 2017, 22, 1250-1273) describe biomarkers directed to suicidality. Weber et al. (Arthritis Research and Therapy, 2016, 18, 1-14) disclose serum cytokines and metalloproteinases as biomarkers for disc herniation, spinal stenosis or degenerative disc disease. Fordyce et al. (Journal of the Neurological Sciences, 2008, Vol. 271, Issue 1-2, 80-86) describe TNFα, IL-1β, IL-6 and leptin as biomarkers in post-poliomyelitis syndrome. Jin et al. (PLOS ONE, 2013, 8(11)) disclose major histocompatibility complex class I A subtype (*HLA-A29.1*), matrix metalloproteinase 9 (*MMP9*), alanine aminopeptidase N (*ANPEP*), L-histidine decarboxylase (HDC), granulocyte colony-stimulating factor 3 receptor (*G*-*CSF3R*), and signal transducer and activator of transcription 3 (*STAT3*) as biomarkers for the Complex regional pain syndrome (CRPS). Luchtin et al. (Biomarker Medicine, 2017, 11(3), 265-276) describe the soluble intercellular adhesion molecule-1 (sICAM-1) as biomarker for pain intensity in chronic pain. Schistad et al. (European Journal of Pain, 2014, 18, 1394-1401) disclose IL-6 as biomarker for 1-year recovery in lumbar radicular pain and Kraychete et al. (Rev Bras Anestesiol, 2009, 59(3), 297-303) describe proinflammatory cytokines an important role in pathophysiology of neuropathic pain syndromes.

Pain is a subjective sensation that reflects bodily damage and the possibility of future harm. Pain treatment is a multi-billion dollar market in the United States. The United States is, however, experiencing an opioid abuse epidemic.

Mental states can affect the perception of pain, and in turn, can be affected by pain. Psychiatric patients may have an increased perception of pain, as well as increased physical health reasons for pain due to their often adverse life trajectory.

Currently, there are no objective tests for determining pain, so clinicians must rely on self-reporting by patients. An objective test for pain can facilitate proper diagnosis and treatment, enabling more confident treatment for those needing treatment for pain, and avoid overprescribing of potentially addictive medications to those not in need. Blood biomarkers for pain can serve as companion diagnostics for clinical trials for the development of new pain medications and repurposing existing drugs for use as pain treatments. Accordingly, there exists a need for objective measures for determining pain, which can guide appropriate treatment.

### SUMMARY OF THE DISCLOSURE

The present invention is defined in the claims. The present disclosure relates generally to methods for determining pain. More particularly, the present disclosure relates to methods for objectively determining pain intensity, predicting future emergency department (ED) visits for pain. Also disclosed are methods for identifying drug and natural compounds as candidates for treating pain using biomarker gene expression signatures.

In one aspect, the present disclosure is directed to a method for determining pain intensity in a subject in need thereof. The method comprises: obtaining an expression level of a blood biomarker in a sample obtained from the subject; obtaining a reference expression level of a blood biomarker in the sample; and identifying a difference between the expression level of the blood biomarker in a sample obtained from the subject and the reference expression level of a blood biomarker, wherein the difference in the expression level of the blood biomarker in the sample obtained from the subject and the reference expression level of the blood biomarker determines pain intensity. The blood biomarker is a panel of blood biomarkers. The reference level can be an average or reference range in the population (a "cross-sectional" approach), or it can be the level of a sample obtained previously in the subject when the subject was not in need of treating pain (a "longitudinal" approach).

Further, a method for identifying a blood biomarker for pain is described, the method comprising: obtaining a first biological sample from a subject and administering a first pain intensity test to the subject; obtaining a second biological sample from the subject and administering a second pain intensity test to the subject; identifying a first cohort of subjects by identifying subjects having a change from low pain intensity to high pain intensity as determined by a difference between the first pain intensity test and the second pain intensity test; identifying candidate biomarkers in the first cohort by identifying biomarkers having a change in expression between the first biological sample and the second biological sample.

In one aspect, the present disclosure is directed to a method for predicting future emergency department (ED) visits for pain. The method comprises: obtaining an expression level of a blood biomarker or panel of blood biomarkers in a sample obtained from the subject; obtaining a reference expression level of the blood biomarker or panel of blood biomarkers in a sample or literature; identifying a difference in the expression level of the blood biomarkers in the sample and the reference expression level of the blood biomarkers; wherein the difference in the expression level of the blood biomarkers in the sample obtained from the subject and the reference expression level of the blood biomarkers determines the likelihood of future ED visits for pain. In one embodiment, the blood biomarker is a panel of blood biomarkers. The reference expression level can be that as described herein.

In addition, a method for mitigating pain in a subject in need thereof is described. The method comprises: obtaining an expression level of a blood biomarker in a sample obtained from the subject; obtaining a reference expression level of the blood biomarker in a sample or literature; identifying a difference in the expression level of the blood biomarker in the sample and the reference expression level of the blood biomarker; afterwards a treatment is administered, wherein the treatment reduces the difference between the expression level of the blood biomarker in the sample and the reference expression level of the blood biomarker to mitigate pain in the subject. The blood biomarker is a panel of blood biomarkers. The reference expression level can be that as described herein.

### BRIEF DESCRIPTION OF THE DRAWINGS

The disclosure will be better understood, and features, aspects and advantages other than those set forth above will become apparent when consideration is given to the following detailed description thereof. Such detailed description makes reference to the following drawings, wherein:

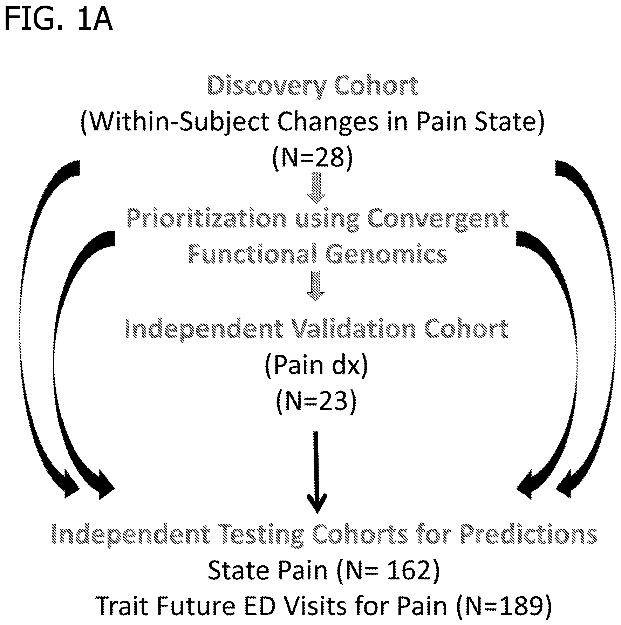

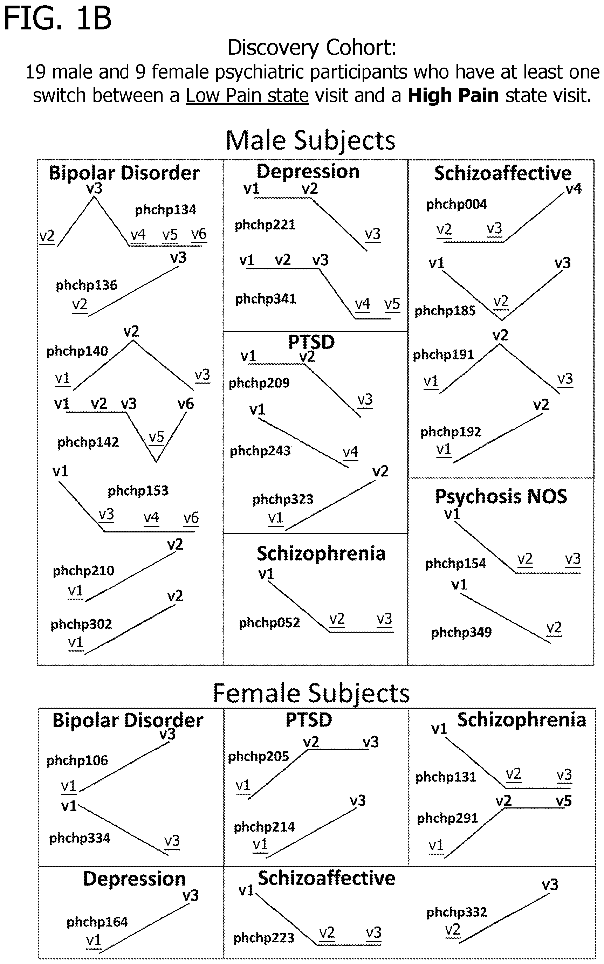

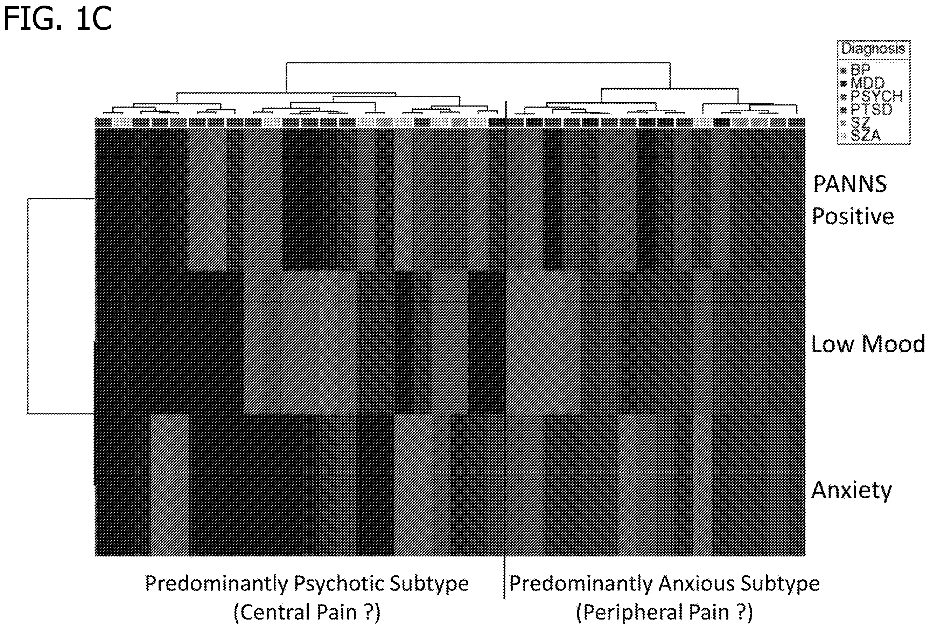

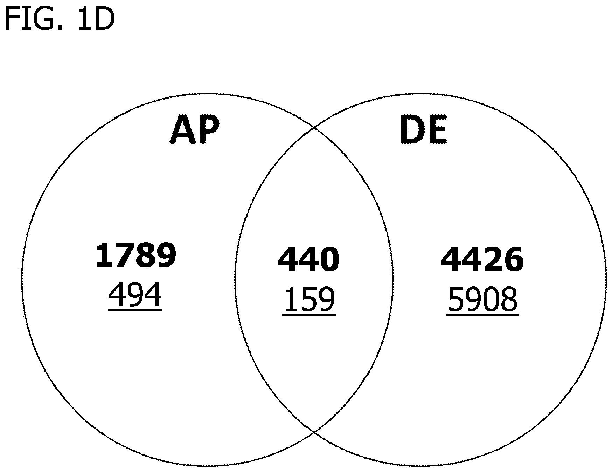

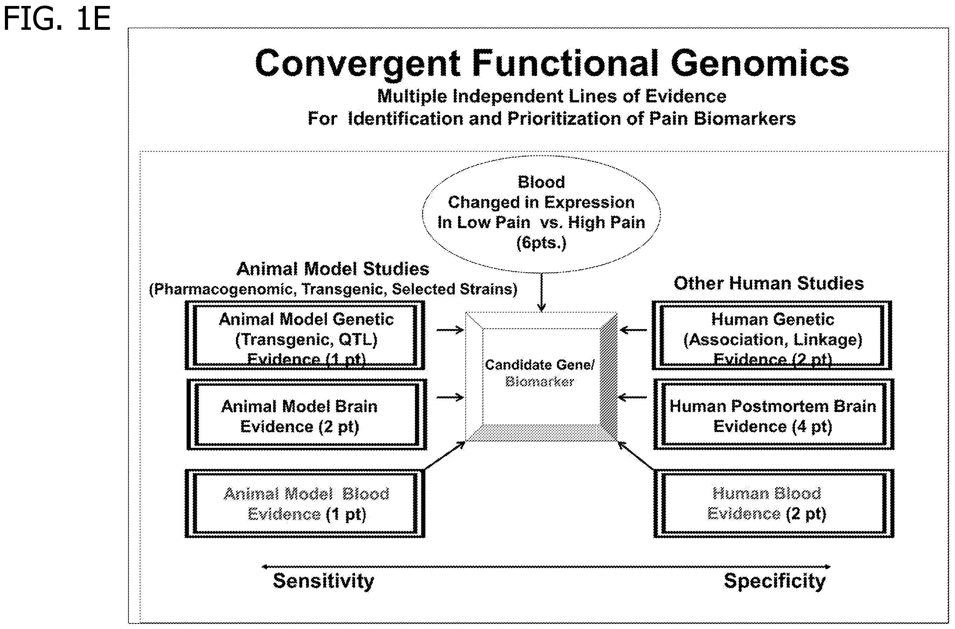

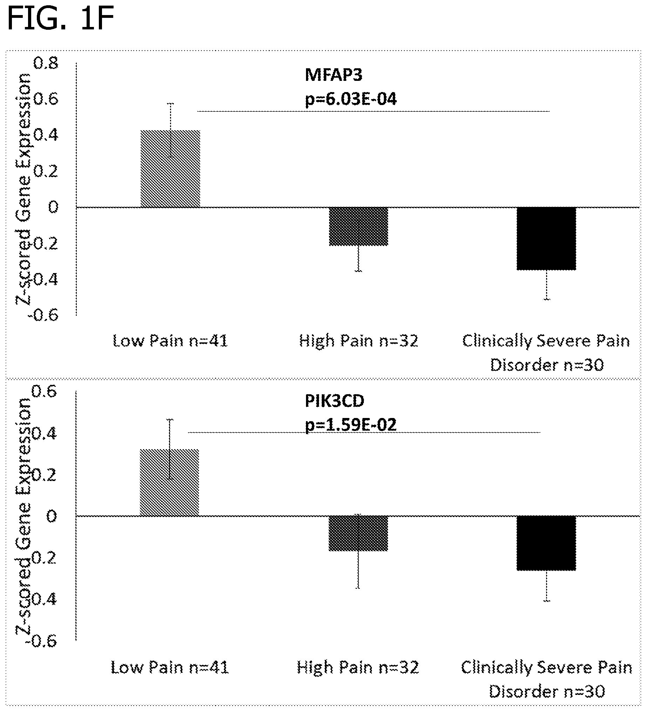

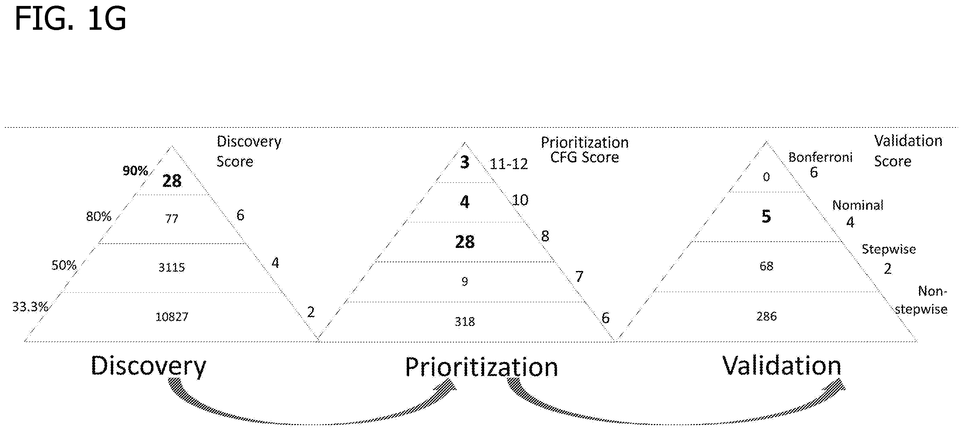

FIGS. 1A-1G depict Steps 1-3: Discovery, Prioritization and Validation. FIG. 1A depicts Cohorts used in study, depicting flow of discovery, prioritization, and validation of biomarkers from each step. FIG. 1B depicts Discovery cohort longitudinal within-participant analysis. Phchp### is study ID for each participant. V# denotes visit number. FIG. 1C depicts Discovery of possible subtypes of Pain based on High Pain visits in the discovery cohort. Participants were clustered using measures of mood and anxiety (Simplified Affective State Scale (SASS)), as well as psychosis (PANNS Positive) FIG. 1D depicts Differential gene expression in the Discovery cohort -number of genes identified with differential expression (DE) and absent-present (AP) methods with an internal score of 1 and above. Red/Underlined-increased in expression in High Pain, blue/Bold-decreased in expression in High Pain. At the discovery step probesets are identified based on their score for tracking pain with a maximum of internal points of 6 (33% (2pt), 50% (4pt) and 80% (6pt)). FIG. 1E depicts prioritization with CFG for prior evidence of involvement in pain. In the prioritization step probesets are converted to their associated genes using Affymetrix annotation and GeneCards. Genes are prioritized and scored using CFG for pain evidence with a maximum of 12 external points. Genes scoring at least 6 points out of a maximum possible of 18 total internal and external scores points are carried to the validation step. FIG. 1F depicts Validation in an independent cohort of psychiatric patients with co-morbid pain disorders and severe subjective and functional pain ratings. In the validation step biomarkers are assessed for stepwise change from the discovery groups of participants with Low Pain, to High Pain, to Clinically Severe Pain disorder, using ANOVA. N= number of testing visits. 5 biomarkers were nominally significant, MFAP3 and PIK3CD were the most significant, and 68 biomarkers were stepwise changed.

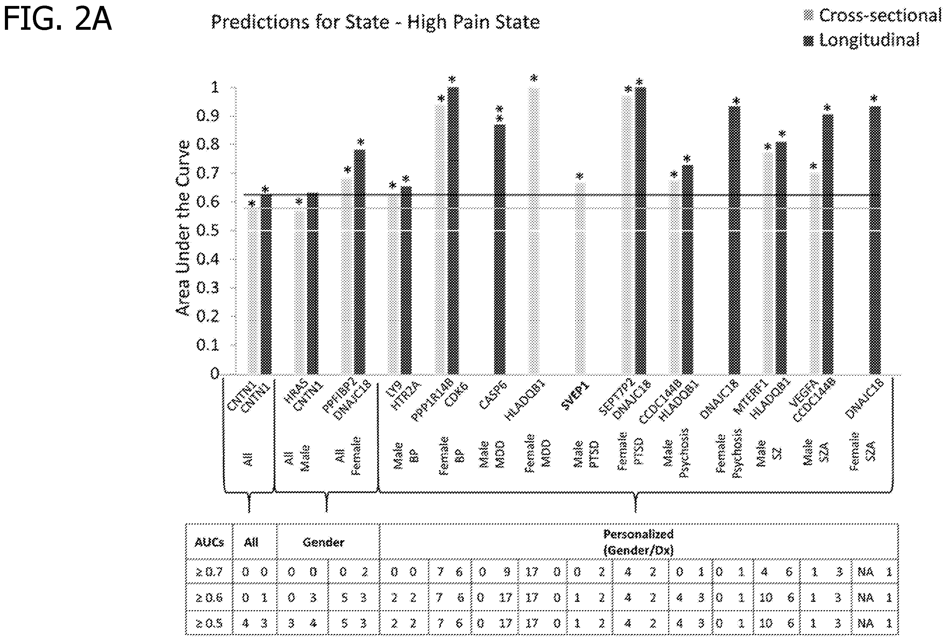

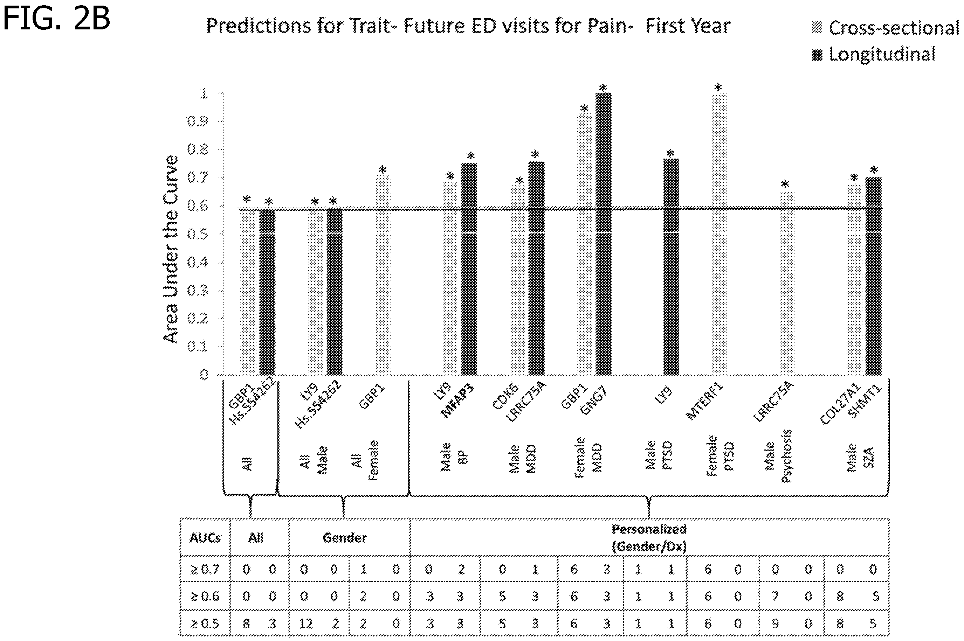

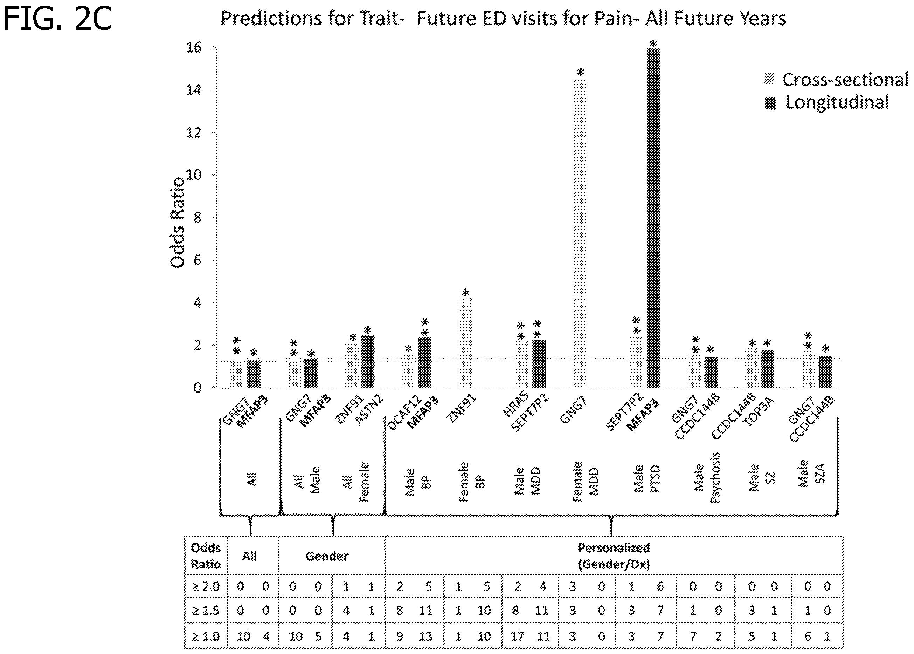

FIGS. 2A-2C depict Best Single Biomarkers Predictors for State Predictions (FIG. 2A), Trait Predictions First Year (FIG. 2B), and Trait Predictions All Future Years (FIG. 2C). From the long list (n=65). Those on short list (n= 5) are bolded. Bar graph shows best predictive biomarkers in each group. * Nominally significant p<0.05. ** Bonferroni significant for the 65 biomarkers tested. Table underneath the figures displays the actual number of biomarkers for each group whose ROC AUC p-values were at least nominally significant. Some female diagnostic groups were omitted from the graph as they did not have any significant biomarkers. Cross-sectional was based on levels at one visit. Longitudinal was based on levels at multiple visits (integrates levels at most recent visit, maximum levels, slope into most recent visit, and maximum slope). Dividing lines represent the cutoffs for a test performing at chance levels (white), and at the same level as the best biomarkers for all subjects in cross-sectional (gray) and longitudinal (black) based predictions. All biomarkers performed better than chance. Biomarkers also performed better when personalized by gender and diagnosis.

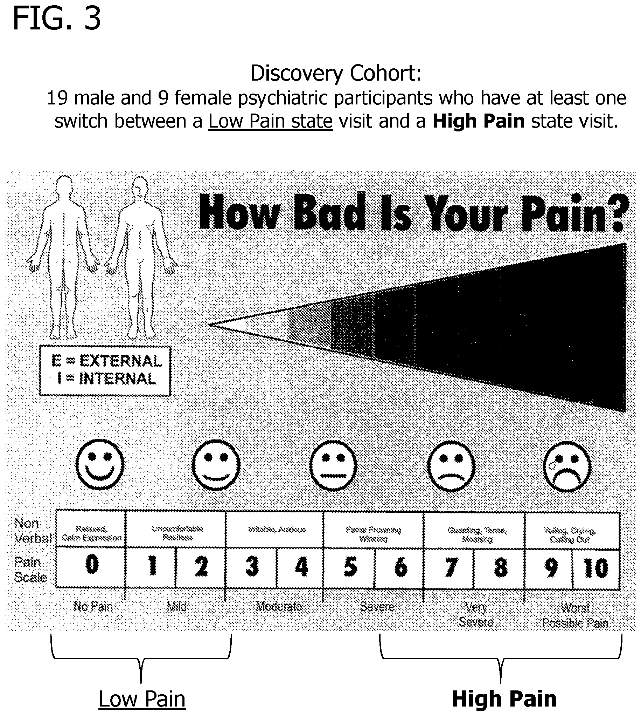

FIG. 3 depicts the pain scale of male and female psychiatric participants.

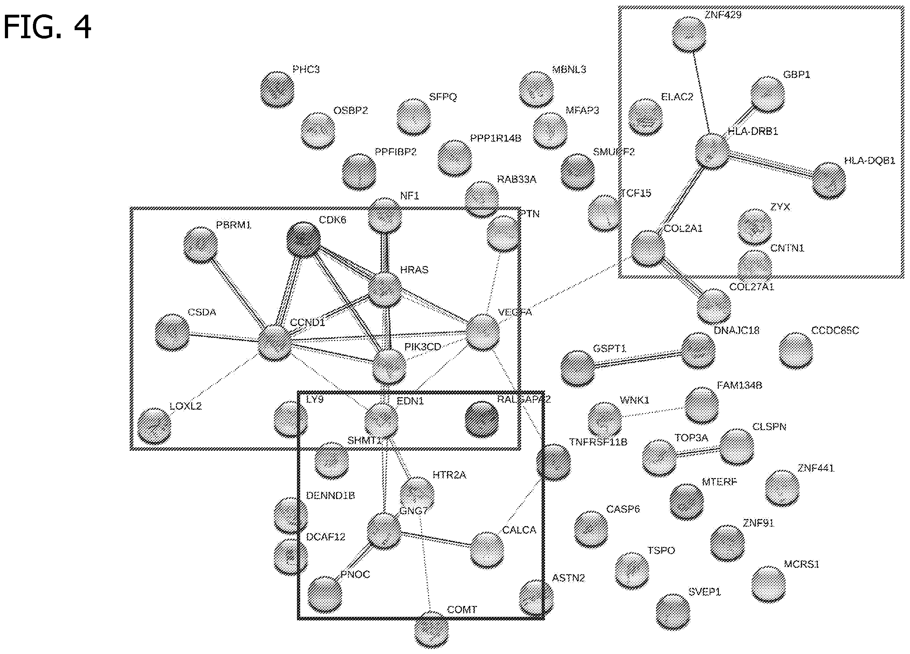

FIG. 4 depicts the STRING interaction network for 60 top biomarkers for pain.

While the disclosure is susceptible to various modifications and alternative forms, specific embodiments thereof have been shown by way of example in the drawings and are herein described below in detail. It should be understood, however, that the description of specific embodiments is not intended to limit the disclosure to cover all modifications, equivalents and alternatives falling within the spirit and scope of the disclosure as defined by the appended claims.

### DETAILED DESCRIPTION

Unless defined otherwise, all technical and scientific terms used herein have the same meaning as commonly understood by one of ordinary skill in the art to which the disclosure belongs. Although any methods and materials similar to or equivalent to those described herein may be used in the practice or testing of the present disclosure, the preferred materials and methods are described below.

In accordance with the present disclosure, methods have been developed to objectively determine pain intensity and predict future emergency department (ED) visits for pain.

In some embodiments, the methods of the present disclosure as described herein are intended to include the use of such methods in "at risk" subjects, including subjects unaffected by or not otherwise afflicted with pain as described herein, for the purpose of diagnosing, prognosing and identifying subjects such that treatment, treatment planning, and treatment options for pain can be made. As used herein, a subject "at risk for pain" refers to individuals who may develop pain. As such, in some embodiments, the methods disclosed herein are directed to a subset of the general population such that, in these embodiments, not all of the general population may benefit from the methods. Based on the foregoing, because some of the method embodiments of the present disclosure are directed to specific subsets or subclasses of identified subjects (that is, the subset or subclass of subjects "at risk for" the specific conditions noted herein), not all subjects will fall within the subset or subclass of subjects as described herein.

Particularly suitable subjects are humans. Suitable subjects can also be experimental animals such as, for example, monkeys and rodents, that display a behavioral phenotype associated with pain. In one particular aspect, the subject is a female human. In another particular aspect, the subject is a male human.

Suitable samples can be, for example, saliva, blood, plasma, serum and a cheek swab. The samples can be further processed using methods known to those skilled in the art to isolate molecules contained in the sample such as, for example, cells, proteins and nucleic acids (e.g., DNA and RNA).

The isolated molecules can also be further processed. For example, cells can be lysed and subjected to methods for isolating proteins and/or nucleic acids contained within the cells. Proteins and nucleic acids contained in the sample and/or in isolated cells can be processed. For example, proteins can be processed for electrophoresis, Western blot analysis, immunoprecipitation and combinations thereof. Nucleic acids can be processed, for example, for polymerase chain reaction, electrophoresis, Northern blot analysis, Southern blot analysis, RNase protection assays, microarrays, serial analysis of gene expression (SAGE) and combinations thereof.

Suitable probes are described herein and can include, for example, nucleic acid probes, antibody probes, and chemical probes.

In some embodiments, the probe can be a labeled probe. Suitable labels can be, for example, a fluorescent label, an enzyme label, a radioactive label, a chemical label, and combinations thereof. Suitable radioactive labels are known to those skilled in the art and can be a radioisotope such as, for example, ³²P, ³³P, ³⁵S, ³H and ¹²⁵I. Suitable enzyme labels can be, for example, colorimetric labels and chemiluminescence labels. Suitable colorimetric (chromogenic) labels can be, for example, alkaline phosphatase, horse radish peroxidase, biotin and digoxigenin. Biotin can be detected using, for example, an anti-biotin antibody, or by streptavidin or avidin or a derivative thereof which retains biotin binding activity conjugated to a chromogenic enzyme such as, for example, alkaline phosphatase and horse radish peroxidase. Digoxigenin can be detected using, for example, an anti-digoxigenin antibody conjugated to a chromogenic enzyme such as, for example, alkaline phosphatase and horse radish peroxidase. Chemiluminescence labels can be, for example, alkaline phosphatase, glucose-6-phosphate dehydrogenase, horseradish peroxidase, Renilla luciferase, and xanthine oxidase. A particularly suitable label can be, for example, SYBR^{®} Green (commercially available from Life Technologies). A particularly suitable probe can be, for example, an oligonucleotide labelled with SYBR^{®} Green. Suitable chemical labels can be, for example, periodate and 1-Ethyl-3-[3-dimethylaminopropyl]carbodiimide hydrochloride (EDC).

As used herein, "diagnosing" and "diagnosis" are used according to their ordinary meaning as understood by those skilled in the art to refer to determining objectively that a subject has increased pain intensity.

As used herein, "predicting pain in a subject in need thereof' refers to indicating in advance that a subject is likely to develop or is at risk for developing pain and/or identifying that a subject with pain wherein the pain is likely to increase and/or identifying a subject that will visit a hospital or other medical facility because of pain and/or because of increasing pain.

As used herein, the term "biomarker" refers to a molecule to be used for analyzing a subject's test sample. Such biomarkers are nucleic acids (such as, for example, a gene, DNA and RNA), proteins and polypeptides. In particular, the biomarker is the levels of expression of a biomarker gene. Suitable biomarker genes are those listed in Tables 1, 4, 5, 7 and combinations thereof.

As used herein, "a reference expression level of a biomarker" refers to the expression level of a biomarker established in a sample for a subject with no pain, expression level of a biomarker in a normal/healthy subject with no pain as determined by one skilled in the art using established methods as described herein, and/or a known expression level of a biomarker obtained from literature. In one suitable embodiment, the reference level can be an average or reference range in the population (a "cross-sectional" approach). In another embodiment, the reference expression level can be the level of a sample obtained previously in the subject when the subject was not in need of treating pain (a "longitudinal" approach). The reference expression level of the biomarker can further refer to the expression level of the biomarker established for a High Pain subject, including a population of High Pain subjects. The reference expression level of the biomarker can also refer to the expression level of the biomarker established for a Low Pain subject, including a population of Low Pain subjects. The reference expression level of the biomarker can also refer to the expression level of the biomarker established for any combination of subjects such as a subject with no pain in a sample, expression level of the biomarker in a sample of a normal/healthy subj ect with no pain, expression level of the biomarker in a sample for a subj ect who has pain at the time the sample is obtained from the subject, but who later exhibits increase in pain, expression level of the biomarker as established for a High Pain subject, including a population of High Pain subjects, and expression level of the biomarker can also refer to the expression level of the biomarker established for a Low Pain subject, including a population of Low Pain subjects. The reference expression level of the biomarker can also refer to the expression level of the biomarker in a sample obtained from the subject to which the method is applied. As such, the change within a subject from visit to visit can indicate increased or decreased pain. For example, a plurality of expression levels of a biomarker can be obtained from a plurality of samples obtained from the same subject and used to identify differences between the plurality of expression levels in each sample. Thus, in some embodiments, two or more samples obtained from the same subject can provide an expression level(s) of a blood biomarker and a reference expression level(s) of the blood biomarker.

As used herein, "expression level of a biomarker" refers to the process by which a gene product is synthesized from a gene encoding the biomarker as known by those skilled in the art. The gene product can be, for example, RNA (ribonucleic acid) and protein. Expression level can be quantitatively measured by methods known by those skilled in the art such as, for example, northern blotting, amplification, polymerase chain reaction, microarray analysis, tag-based technologies (e.g., serial analysis of gene expression and next generation sequencing such as whole transcriptome shotgun sequencing or RNA-Seq), Western blotting, enzyme linked immunosorbent assay (ELISA), and combinations thereof.

As used herein, a "difference" and/or "change" in the expression level of the biomarker refers to an increase or a decrease in the measured expression level of a blood biomarker when analyzed against a reference expression level of the biomarker. In some embodiments, the "difference" and/or "change" refers to an increase or a decrease by about 1.2-fold or greater in the expression level of the biomarker as identified between a sample obtained from the subject and the reference expression level of the biomarker. In one embodiment, the difference and/or change in expression level is an increase or decrease by about 1.2 fold. As used herein "a risk for pain" can refer to an increased (greater) risk that a subject will experience (or develop) pain. For example, depending on the biomarker(s) selected, the difference and/or change in the expression level of the biomarker(s) can indicate an increased (greater) risk that a subject will experience (or develop) pain. Conversely, depending on the biomarker(s) selected, the difference and/or change in the expression level of the biomarker(s) can indicate a decreased (lower) risk that a subject will experience (or develop) pain.

### Biomarkers for use in Pain Treatment

In one aspect, the present disclosure is directed to biomarkers for use in a method for treating pain in a subject in need thereof. The method includes: obtaining an expression level of a blood biomarker in a sample obtained from the subject; obtaining a reference expression level of the blood biomarker in a sample or literature; identifying a difference in the expression level of the blood biomarker in the sample and the reference expression level of the blood biomarker; and administering a treatment, wherein the treatment reduces the difference between the expression level of the blood biomarker in the sample and the reference expression level of the blood biomarker to mitigate pain in the subject.

The biomarkers are selected from the group listed in Table 1. A panel of blood biomarkers is used. Biomarkers can be selected with different weighting coefficients possible.

Suitable treatments include those listed in Tables 1, 2, 7, and combinations thereof. Suitable treatments further include pain treatments known to those skilled in the art. Particularly suitable treatments include SC-560, pyridoxine, methylergometrine, LY-294002, haloperidol, cytisine, cyanocobalamin, apigenin, betaescin, amoxapine, and combinations thereof.

In some embodiments, the expression level of the blood biomarker in the sample obtained from the subject is decreased as compared to the reference expression level of the biomarker.

In some embodiments, the expression level of the blood biomarker in the sample obtained from the subject is increased as compared to the reference expression level of the biomarker.

The method further includes performing a neuropsychological test on the subject. Generally, neuropsychological testing includes a comprehensive assessment of cognitive and personality functioning. More particularly, exemplary neuropsychological tests include: for intelligence (e.g., WAIS, WISC, SB, TONI); for achievement (e.g., WJ-III, WIAT, WRAT); for attention (e.g., CCPT, WCST, Vanderbilt, NEPSY); for language (e.g., GORT, Boston Naming, HRB-Aphasia for memory and learning (e.g., WMS, WRAML, CVLT, RAVLT, ROCF, NEPSY); for motor control (e.g., Grooved Pegoard, Finger Tapping, Grip Strength, Lateral Dominance); for visual (e.g., Spatial - ROCFT, Bender-Gestalt, HVOT); for autism (e.g., ADOS, ASDS, ADI, GARS); for executive functioning (e.g., WCST, BRIEF, EFSD, D-KEFS, HRB); and for behavioral (e.g., BASC, Achenbach, Vanderbilt).

### Methods for Determining Pain

A method for determining High Pain intensity in a subject in need thereof is described. The method includes: obtaining an expression level of a blood biomarker in a sample obtained from the subject; obtaining a reference expression level of the blood biomarker; and identifying a difference in the expression level of the blood biomarker in the sample and the reference expression level of the blood biomarker.

As described herein, "Low Pain" refers to Visual Analog Scale (VAS) for pain of 2 and below; "Intermediate Pain" refers to VAS of 3-5; and "High Pain" refers to VAS of 6 and above (see, FIG. 3). The pain VAS is self-completed by the subject. The pain VAS is a continuous scale comprised of a horizontal (HVAS) or vertical (VVAS) line, usually 10 centimeters (100 mm) in length, anchored by 2 verbal descriptors, one for each symptom extreme (at 0 for "no pain" and at 100 for "worst imaginable pain"). The subject is asked to place a line perpendicular to the VAS line at the point that represents their pain intensity. Using a ruler, the score (i.e., intensity of pain) is determined by measuring the distance (mm) on the 10-cm line between the "no pain" anchor and the patient's mark, providing a range of scores from 0-100. A higher score indicates greater pain intensity.

While not used herein, other suitable pain tests include, for example, numeric rating scale (NRS), McGill Pain Questionnaire (MPQ), Short-form McGill Pain Questionnaire (SF-MPQ), Chronis Pain Grade Scale (CPGS), Short form 36 Bodily Pain Scale (SF-36 BPS), Measure of Intermittent and Constant Osteoarthritis Pain (ICOAP), and combinations thereof. For more information on these tests and applications thereof, *see* Hawker et al., Arthritis Care & Research, vol. 36, no. S11, November 2011, pp. S240-S252.

The biomarkers are selected from the group listed in Table 1. Further described are biomarkers of Tables 4, 5, 7 and combinations thereof. A panel of blood biomarkers is used. Biomarkers can be selected with different weighting coefficients possible.

In some embodiments, the expression level of the blood biomarker in the sample obtained from the subject is increased as compared to the reference expression level of the biomarker.

In some embodiments, the expression level of the blood biomarker in the sample obtained from the subject is decreased as compared to the reference expression level of the biomarker.

A particularly suitable biomarker for determining pain intensity is CNTN1.

In some embodiments, the subject is a female. A particularly suitable biomarker for predicting pain state in female subjects is DNAJC18.

A particularly suitable biomarker for predicting pain state in female subjects is CTN1.

In some embodiments, the method further includes performing a neuropsychological test on the subject.

### Methods for Predicting Future Medical Care Facility Visit for Pain

In another aspect, the present disclosure is directed to biomarkers for use in a method for predicting a future medical care facility visit for pain in a subject in need thereof. The method includes: obtaining an expression level of a blood biomarker in a sample obtained from the subject; obtaining a reference expression level of the blood biomarker; and identifying a difference in the expression level of the blood biomarker in the sample and the reference expression level of the blood biomarker, whereas the difference in the expression level of the blood biomarker in the sample obtained from the subject and the reference expression level of the blood biomarker determines the likelihood of future medical care facility/emergency department (ED) visits for pain.

As used herein, "emergency department (ED)" is used according to its ordinary meaning as understood by those skilled in the art to refer to medical care facilities specializing in emergency medicine, the acute care of patients who present without prior appointment; either by their own means or by that of an ambulance, and includes accident & emergency departments (A&E), emergency rooms (ER), emergency wards (EW) and casualty departments.

The biomarker is selected from the group listed in Table 1. Further described are biomarkers of Tables 4, 5, 7 and combinations thereof. A panel of blood biomarkers is used. Biomarkers can be selected with different weighting coefficients possible.

In some embodiments, the expression level of the blood biomarker in the sample obtained from the subject is increased as compared to the reference expression level of the biomarker.

In some embodiments, the expression level of the blood biomarker in the sample obtained from the subject is decreased as compared to the reference expression level of the biomarker.

GBP1 is particularly suitable for predicting trait first year ED visits. GNG7 is particularly suitable for predicting trait all future ED visits.

In some embodiments, the subject is a female. GBP1 is particularly suitable as a predictor for trait first year ED visits in female subjects. ASTN2 is particularly suitable for trait all future ED visits in female subjects. When the subject is a female with bipolar disorder, CDK6 is a particularly suitable predictor for state. When the subject is a female with PTSD, SHMT1 is a particularly suitable predictor for trait first year ED visits. When the subject is a female with depression, GNG7 is a particularly suitable for trait all future ED visits.

In some embodiments, the subject is a male. CTN1 is particularly suitable as a predictor for state in male subjects. Hs.554262 is particularly suitable as a predictor for trait first year ED visits in male subjects. MFAP3 is particularly suitable for trait all future ED visits in male subjects. When the subject is a male with depression, CASPS is particularly suitable as a predictor for state. When the subject is a male with PTSD, LY9 is particularly suitable as a strong predictor for trait first year ED visits. When the subject is a male with PTSD MFAP3 is particularly suitable as a strong predictor for trait all future ED visits.

Particularly suitable biomarkers for pain include CCDC144B (Coiled-Coil Domain Containing 144B), COL2A1 (Collagen Type II Alpha 1 Chain), PPFIBP2 (PPFIA Binding Protein 2), DENND1B (DENN Domain Containing 1B), ZNF441 (Zinc Finger Protein 441), TOPS A (Topoisomerase (DNA) III Alpha), and ZNF429 (Zinc Finger Protein 429), and combinations thereof.

The method further includes performing a neuropsychological test on the subject.

### Prognosis of Pain

In another aspect, the present disclosure is directed to a method of prognosing pain in an individual in need thereof. As used herein, the term "prognosing" and "prognosis" are used according to their ordinary meaning as understood by those skilled in the art to refer to pain level increases from no pain to Low Pain to Moderate (Intermediate) Pain to High Pain.

The method includes: obtaining an expression level of a blood biomarker in a sample obtained from the subject; obtaining a reference expression level of the blood biomarker; and identifying a difference in the expression level of the blood biomarker in the sample and the reference expression level of the blood biomarker.

The method further includes performing a neuropsychological test on the subject.

### EXAMPLES

### Materials and Methods

Three independent cohorts were used: discovery (major psychiatric disorders), validation (major psychiatric disorders with clinically severe pain disorders), and testing (an independent major psychiatric disorders cohort for predicting pain state, and for predicting future ER visits for pain) (see, FIG. 1A)

The psychiatric participants/subjects were part of a larger longitudinal cohort of adults that are being continuously collected. Participants were recruited from the patient population at the Indianapolis VA Medical Center. All participants understood and signed informed consent forms detailing the research goals, procedure, caveats and safeguards, per IRB approved protocol. Participants completed diagnostic assessments by an extensive structured clinical interview - Diagnostic Interview for Genetic Studies, and up to six testing visits, 3-6 months apart or whenever a new psychiatric hospitalization occurred. At each testing visit, the subject received a series of rating scales, including a visual analog scale (1-10) for assessing pain and the SF-36 quality of life scale, which has two pain related items (items 21 and 22), and blood was drawn. Whole blood (10 ml) was collected in two RNA-stabilizing PAXgene tubes, labeled with an anonymized ID number, and stored at -80 °C in a locked freezer until the time of future processing. Whole-blood RNA was extracted for microarray gene expression studies from the PAXgene tubes, as detailed below.

For these Examples, the within-participant discovery cohort, from which the biomarker data were derived, consisted of 28 participants (19 males, 9 females) with multiple testing visits, who each had at least one diametric change in pain from Low Pain (VAS of 2 and below) to High Pain (VAS of 6 and above) from one testing visit to another (FIGS. 1B and 3). There were 3 participants with 5 visits each, 1 participants with 4 visits each, 12 participants with 3 visits each, and 12 participants with 2 visits each resulting in a total of 79 blood samples for subsequent gene expression microarray studies (FIGS. 1A-1C; Table 3).

The validation cohort, in which the top biomarker findings were validated for being even more changed in expression, consisted of 13 male and 10 female participants with a pain disorder diagnosis and clinically severe pain (Table 3). This was determined as having a pain VAS of 6 and above and a sum of SF36 scale items 21 (pain intensity) and 22 (impairment by pain of daily activities) of 10 and above. (*See*, Table 3).

The independent test cohort for predicting state (High Pain) consisted of 134 male and 28 female participants with psychiatric disorders, demographically matched with the discovery cohort, with one or multiple testing visits, with either Low Pain, intermediate Pain, or High Pain, resulting in a total of 414 blood samples in which whole-genome blood gene expression data were obtained (FIGS. 1A-1C and Table 3).

The test cohort for predicting trait (future ED visits with pain as the primary reason in the first year of follow-up, and all future ED visits for pain) (FIGS. 1A-1C) consisted of 171 males and 19 female participants for which longitudinal follow-up with electronic medical records were obtained. The participants' subsequent number of ED pain-related visits in the year following testing was tabulated from electronic medical records by a clinical researcher, who used the key word "pain" in the reasons for ED visit, or "ache" with a mention of acute pain in the text of the note.

Medications. The participants in the discovery cohort were all diagnosed with various psychiatric disorders, and had various medical co-morbidities (Table 1). Their medications were listed in their electronic medical records, and documented at the time of each testing visit. Medications can have a strong influence on gene expression. However, the discovery of differentially expressed genes was based on within-participant analyses, which factored out not only genetic background effects, but also minimizes medication effects, as the participants rarely had major medication changes between visits. Moreover, there was no consistent pattern of any particular type of medication, as the participants were on a wide variety of different medications, psychiatric and non-psychiatric. Some participants may be non-compliant with their treatment and may thus have changes in medications or drug of abuse not reflected in their medical records. That being said, the goal was to discover biomarkers that track pain, regardless if the reason for it was endogenous biology or driven by substance abuse or medication non-compliance. In fact, one would expect some of these biomarkers to be targets of medications. Overall, the discovery of biomarkers with the universal design occurred despite the participants having different genders, diagnoses, being on various different medications, and other lifestyle variables.

### Blood Gene Expression Experiments

RNA extraction. Whole blood (2.5-5 ml) was collected into each PaxGene tube by routine venipuncture. RNA was extracted and processed as previously described (see, Le-Niculescu, H. et al. Mol Psychiatry 18, 1249-64 (2013); Niculescu, A.B. et al. Mol Psychiatry 20, 1266-85 (2015); Levey, D.F. et al. Mol Psychiatry 21, 768-85 (2016)).

Microarrays. Microarray work was carried out as previously described (see, Le-Niculescu, H. et al. Mol Psychiatry 18, 1249-64 (2013); Niculescu, A.B. et al. Mol Psychiatry 20, 1266-85 (2015); Levey, D.F. et al. Mol Psychiatry 21, 768-85 (2016)).

### Biomarkers

### Step 1: Discovery.

The participant's score from the VAS Pain Scale was used, assessed at the time of blood collection (FIGS. 1A-1C). Gene expression differences between visits were analyzed with Low Pain (defined as a score of 0-2) and visits with High Pain (defined as a score of 6 and above), using a powerful within-participant design, then an across-participants summation (FIGS. 1A-1C).

Data was analyzed using an Absent-Present (AP) approach and a differential expression (DE) approach (see, Le-Niculescu, H. et al. Mol Psychiatry 18, 1249-64 (2013); Niculescu, A.B. et al. Mol Psychiatry 20, 1266-85 (2015); Levey, D.F. et al. Mol Psychiatry 21, 768-85 (2016)). The AP approach can capture turning on and off of genes, and the DE approach can capture gradual changes in expression. R scripts were developed to automate and conduct all these large dataset analyses in bulk, checked against human manual scoring.

Gene symbol for the probe sets were identified using NetAffyx (Affymetrix) for Affymetrix HG-U133 Plus 2.0 GeneChips, followed by GeneCards to confirm the primary gene symbol. For those probesets that were not assigned a gene symbol by NettAffyx, GeneAnnot was used to obtain gene symbols for the uncharacterized probesets, followed by GeneCard. Genes were then scored using a manually curated CFG database as described below (FIG. 1E).

### Step 2. Prioritization using Convergent Functional Genomics (CFG).

Databases. Manually curated databases of the human gene expression/protein expression studies (postmortem brain, peripheral tissue/fluids: CSF, blood and cell cultures), human genetic studies (association, copy number variations and linkage), and animal model gene expression and genetic studies, published to date on psychiatric disorders, were created. Only findings deemed significant in the primary publication, by the study authors, using their particular experimental design and thresholds were included in the databases. The databases included only primary literature data and did not include review papers or other secondary data integration analyses to avoid redundancy and circularity. These large and constantly updated databases have been used in the inventors' CFG cross validation and prioritization platform (FIG. 1E). For these Examples, data from 355 papers on pain were present in the databases at the time of the CFG analyses (December 2017) (human genetic studies-212, human nervous tissue studies-3, human peripheral tissue/fluids- 57, non-human genetic studies-26, non-human brain/nervous tissue studies-48, non-human peripheral tissue/fluids- 9). Analyses were performed as described herein and in Le-Niculescu, H. et al. Mol Psychiatry 18, 1249-64 (2013); Niculescu, A.B. et al. Mol Psychiatry 20, 1266-85 (2015); Levey, D.F. et al. Mol Psychiatry 21, 768-85 (2016).

### Step 3. Validation analysis.

Validation analyses of candidate biomarker genes were conducted separately for AP and for DE. Which of the top candidate genes (total CFG score of 6 or above), were stepwise changed in expression from the Low Pain and High Pain group to the Clinically Severe Pain group was determined. A CFG score of 6 or above reflected an empirical cutoff of 33.3% of the maximum possible CFG score of 12, which permitted the inclusion of potentially novel genes with maximal internal score of 6 but no external evidence score. Participants with Low Pain, as well as participants with High Pain from the discovery cohort who did not have severe clinical pain (SF36 sum of item 21 and 22 <10) were used, along with the independent validation cohort which all had severe clinical pain and a co-morbid pain disorder diagnosis (n= 23).

For the AP analysis, the Affymetrix microarray .chp data files from the participants in the validation cohort of severe pain were imported into MAS5 Affymetrix Expression Console, alongside the data files from the Low Pain and High Pain groups in the live discovery cohort. The AP data was transferred to an Excel sheet and A was transformed into 0, M into 0.5 and P into 1. Everything was Z-scored together by gender and diagnosis. If a probe set would have shown no variance, and thus, gave a non-determined (0/0) value in Z-scoring in a gender and diagnosed, the value was excluded from the analysis for that probeset for that gender and diagnosis from the analysis.

For the DE analysis, the cohorts were assembled out of Affymetrix .cel data that was RMA normalized by gender and diagnosis. The log transformed expression data was transferred to an Excel sheet, and non-log data transformed by taking 2 to the power of the transformed expression value. The values were then Z-scored by gender and diagnosis.

The Excel sheets with the Z-scored by gender and diagnosis AP and DE expression data were imported into Partek, and statistical analyses were performed using a one-way ANOVA for the stepwise changed probesets, and a stringent Bonferroni corrections were performed for all the probesets tested in AP and DE (stepwise and non-stepwise) (FIG. 1F). An R script that automatically analyzes the data directly from the Excel sheet was then developed and used to confirm the calculations.

### Choice of biomarkers to be carried forward

The top biomarkers from each step were carried forward. The longer list of candidate biomarkers includes the top biomarkers from discovery step (>=90% of scores, n=28), the top biomarkers from the prioritization step (CFG score >=8, n=32), and the nominally significant biomarkers after the validation step (n=5), for a total of n= 65 probesets (n=60 genes). The short list of top biomarkers after the validation step is 5 biomarkers. In Step 4 testing, prediction with the biomarkers from the long list in independent cohorts High Pain state, and future ED visits for pain in the first year, and in all future years were performed.

### Diagnostics

The test cohort for predicting High Pain (state), and the subset of it that was a test cohort for predicting future ER visits (trait), were assembled out of data that was RMA normalized by gender and diagnosis. The cohort was completely independent, as there was no subject overlap with the discovery cohort. Phenomic (clinical) and gene expression markers used for predictions were Z-scored by gender and diagnosis to be able to combine different markers into panels and to avoid potential artifacts due to different ranges of expression in different gender and diagnoses. Markers were combined by simple summation of the increased risk markers minus the decreased risk markers. Predictions were performed using R studio.

Predicting High Pain State. Receiver-operating characteristic (ROC) analyses between genomic and phenomic marker levels and Pain were performed by assigning participants with a Pain score of 6 and greater into the High Pain category. The pROC package of R (Xavier Robin et al. BMC Bioinformatics 2011) was used. The z-scored biomarker and phene scores were run in the ROC generating program against the diagnostic groups in the independent test cohort (High Pain vs. the rest of participants). Additionally, a one-tailed t-test was performed between High Pain group versus the rest, and Pearson R (one-tail) was calculated between Pain scores and marker levels.

Predicting Future ER visits for Pain in First Year Following Testing. Analysis for predicting ER visits for Pain in the first year following each testing visit in subjects that had at least one year of follow-up in the VA system was conducted. ROC analysis between genomic and phenomic marker levels at specific testing visit and future ER visits for Pain were performed as previously described based on assigning if participants had visited the ER with primary reason for Pain or not within one year following a testing visit. Additionally, a one tailed t-test with unequal variance was performed between groups of participant visits with and without ER visits for pain. Person R (one-tail) correlation was performed between hospitalization frequency (number of ER visits for pain divided by duration of follow-up) and marker levels. A Cox regression was performed using the time in days from the testing visit date to first ER visit date in the case of patients who had been to the ER, or 365 days for those who did not. The hazard ratio was calculated such that a value greater than 1 always indicated increased risk for ER visits, regardless if the biomarker was increased or decreased in expression.

Odds ratio analysis was conducted for ER visits for pain for all future ER visits due to pain, including those occurring beyond one year of follow-up, in the years following testing (on average 5.26 years per participant, range 0.44 to 11.27 years; see Tables 1 and 3), as this calculation, unlike the ROC and t-test, accounts for the actual length of follow-up, which varied from participant to participant. Without being bound by theory, the ROC and t-test may, if used, under-represent the power of the markers to predict, as the more severe psychiatric patients are more likely to move geographically and/or be lost to follow-up. A Cox regression was also performed using the time in days from visit date to first ER Pain visit date in the case of patients who had been to the ER for pain, or from visit date to last note date in the electronic medical records for those who did not. The hazard ration was calculated such that a value greater than 1 always indicated increased risk for ER Pain related visits, regardless if the biomarker was increased or decreased in expression.

### Biological Understanding

### Pathway analysis

IPA (Ingenuity Pathway Analysis, version 24390178, Qiagen), David Functional Annotation Bioinformatics Microarray Analysis (National Institute of Allergy and Infectious Diseases) version 6.7 (August 2016), and Kyoto Encyclopedia of Genes and Genomes (KEGG) (through DAVID) were used to analyze the biological roles, including top canonical pathways and diseases (Table 6), of the candidate genes resulting from these Examples, as well as to identify genes in the dataset that were the target of existing drugs. The pathway analysis for the combined AP and DE probesets identified 60 unique genes (65 probesets). Network analysis of the 60 unique genes was performed using STRING Interaction Network by in putting the genes into the search window and performing Multiple Proteins Homo sapiens analysis.

### CFG beyond Pain: evidence for involvement in other psychiatric and related disorders.

A CGF approach was also used to examine evidence from other psychiatric and related disorders for the list of 65 candidate biomarkers (Table 5).

### Therapeutics

Pharmacogenomics. Which of the individual top biomarkers were analyzed for knowing to be modulated by existing drugs using the CFG databases and using Ingenuity Drugs analysis (Table 7).

New drug discovery/repurposing. Drugs and natural compounds were also analyzed as an opposite match for the gene expression profile of panels of the top biomarkers (n=65) using the Connectivity Map (Broad Institute, MIT) (Table 2). 33 of 65 probesets were present in the HGU-133A array used for the Connectivity Map. The NIH LINCS L1000 database was also used (Table 4).

### Convergent Functional Evidence

All the evidence from discovery (up to 6 points), prioritization (up to 12 points), validation (up to 6 points), testing (state, trait first year ED visits, trait all future ED visits- up to 8 points each if significantly predicts in all participants, 6 points if predicts by gender, 4 points if predicts in gender/diagnosis) were tabulated into a convergent functional evidence score. The total score could be up to 48 points: 36 from this data and 12 from literature data. The data from these Examples were weighed three times as much as the literature data. The Examples highlight, based on the totality of the experimental data and of the evidence in the field to date, biomarkers having all around evidence: those that tracked pain, those that predicted it, those that were reflective of pain and other pathology, and those that were potential drug targets.

Provided herein is a powerful longitudinal within-participant design in individuals with psychiatric disorders to discover blood gene expression changes between self-reported Low Pain and High Pain states (FIGS. 1A-1C). A longitudinal within-participant design is orders of magnitude more powerful than a cross-sectional case-control design. Some of these candidate gene expression biomarkers are increased in expression in High Pain states (being putative risk genes, or "algogenes"), and others are decreased in expression (being putative protective genes, or "pain suppressor genes").

The list of candidate biomarkers was prioritized with a Bayesian-like Convergent Functional Genomics approach, comprehensively integrating previous human and animal model evidence in the field.

The top biomarkers from discovery and prioritization were validated in an independent cohort of psychiatric subjects carrying a diagnosis of a pain disorder and with high scores on pain severity ratings. A list of 65 candidate biomarkers (Tables 1 and 3), including a shorter list of 5 validated biomarkers (MFAP3, PIK3CD, SVEP1, TNFRSF11B, ELAC2) was obtained from the first three steps. The biomarkers with the best evidence after validation were Hs.666804/MFAP3 (p=6.03E-04) and PIK3CD (p=1.59E-02).

The 65 candidate biomarkers were analyzed for predicting pain severity state and future emergency department (ED) visits for pain in another independent cohort of psychiatric subjects. The biomarkers were analyzed in all subjects in the test cohort, as well as by gender and psychiatric diagnosis, which showed increased accuracy, particularly in women (FIG. 2). In general, the longitudinal information was more predictive than the cross-sectional information. Across all participants tested, CNTN1 was the best predictor for state (AUC 63%, p=0.0014), GBP1 the best predictor for trait first year ED visits (AUC 59%, p=0.0035), and GNG7 the best predictor for trait all future ED visits (OR 1.28, p=0.000161, surviving Bonferroni correction for the 65 biomarkers tested). By gender, in females, DNAJC18 was the best predictor for state (AUC 78%, p=0.0049), GBP1 the best predictor for trait first year ED visits (AUC 71%, p=0.043) and ASTN2 for trait all future ED visits (OR 2.45, p=0.043). In males, CNTN1 was the best predictor for state (AUC 63%, p=0.0022), Hs.554262 the best predictor for trait first year ED visits (AUC 59%, p=0.016), and MFAP3 the best predictor for trait all future ED visits (OR 1.34, p=0.014). Personalized by gender and diagnosis, in female bipolar, CDK6 was a strong predictor for state (AUC 100%, p=0.007), in female PTSD, SHMT1 was a strong predictor for trait first year ED visits (AUC 100%, p=0.022), and in female depression GNG7 for trait all future ED visits (OR 14.54, p=0.023). In male depression, CASPS was a strong predictor for state (AUC 87%, p=0.00007, surviving Bonferroni correction for the 65 biomarkers tested), in male PTSD, LY9 was a strong predictor for trait first year ED visits (AUC 77%, p= 0.041), and in male PTSD, MFAP3 was a strong predictor for trait all future ED visits (OR 15.95, p=0.00084). Predictions of future ED visits for pain in the independent cohorts were consistently stronger using biomarkers than clinical phenotypic markers (pain VAS scale, pain items 21 and 22 from SF-36), supporting the utility of biomarkers. Also, in general, panels of all 65 biomarkers or of the 5 validated biomarkers did not work as well as individual biomarkers, particularly when the later are tested by gender and diagnosis, consistent with there being heterogeneity in the population and supporting the need for personalization. The notable exception was predicting all future ED visits for pain, where the panel of 5 validated biomarkers performed better than individual biomarkers.

The biomarkers were further analyzed for involvement in other psychiatric and related disorders (Table 5). A majority of the biomarkers have some evidence in other disorders, whereas a few seemed to be specific for pain, such as CCDC144B (Coiled-Coil Domain Containing 144B), COL2A1 (Collagen Type II Alpha 1 Chain), PPFIBP2 (PPFIA Binding Protein 2), DENND1B (DENN Domain Containing 1B), ZNF441 (Zinc Finger Protein 441), TOPS A (Topoisomerase (DNA) III Alpha), and ZNF429 (Zinc Finger Protein 429). A majority of the biomarkers (50 out of 60 genes, i.e. 83.3%) have prior evidence for involvement in suicide, indicating an extensive molecular co-morbidity between pain and suicide, to go along with the clinical and phenomenological co-morbidity (physical pain, psychic pain). The biological pathways and networks the biomarkers are involved in were analyzed (Table 6 and FIG. 4). There was a network centered on GNG7 (FIG. 4), that may be involved in connectivity/signaling, comprising HTR2A, EDN1, PNOC (involved in pain signaling) and CALCA (involved in Reflex Sympathetic Dystrophy and Complex Regional Pain Syndrome). It was reassuring that PNOC (Prepronociceptin) increased in expression in high pain states, i.e. as an algogene. Given its known roles in pain, it can serve as a *de facto* positive control. A second network was centered on CCND1, may be involved in activity/trophicity, and comprises HRAS, CDK6, PBRM1, CSDA, LOXL2, EDN1, PIK3CD, and VEGFA. A third network was centered on HLA DRB1, may be involved in reactivity/immune response, and comprises GBP1, ZNF429, COL2A1, and HLA DQB1, from the list of 65 top biomarkers.

The biomarkers were analyzed as targets of existing drugs and thus could be used for pharmacogenomics population stratification and measuring of response to treatment (Table 7), as well as used the biomarker gene expression signature to interrogate the Connectivity Map database from Broad/MIT to identify drugs and natural compounds that can be repurposed for treating pain (Table 2). The top drugs identified as potential new pain therapeutic were SC-560, an NSAID, haloperidol, an antipsychotic, and amoxapine, an antidepressant. The top natural compounds were pyridoxine (vitamin B6), cyanocobalamin (vitamin B 12), and apigenin (a plant flavonoid).

The biomarkers with the best overall evidence across the six steps were GNG7, CNTN1, LY9 CCDC144B, GBP1 and MFAP3 (Table 1). GNG7 (G Protein Subunit Gamma 7) was decreased in expression in blood in High Pain states, i.e., it is a pain suppressor gene. There is evidence in other tissues in human studies for involvement in pain (diabetic neuropathy, vertebral disc). GNG7 also has trans-diagnostic evidence for involvement in other psychiatric disorders. It is decreased in expression in mouse brain by alcohol, hallucinogens, and stress, and increased in expression by omega-3 fatty acids. CNTN1 (Contactin 1) was decreased in expression in blood in High Pain states, i.e. it is a pain suppressor gene. Reassuringly, there was convergent evidence in other tissues in human studies for involvement in pain: CNTN1 has also been reported to be decreased in expression in CSF in women with chronic widespread pain (CWP). Anti-contactin 1 autoantibodies, that block/decrease levels of contactin 1, have been described in chronic inflammatory demyelinating polyneuropathy4. CNTN1 has also trans-diagnostic evidence for involvement in psychiatric disorders. It is decreased in expression in schizophrenia brain and blood, and in blood in suicidality in females. CNTN1 was increased in expression by clozapine in mouse brain. LY9 (Lymphocyte Antigen 9) was increased in expression in blood in High Pain states, i.e. it is an algogene. It also has epigenetic evidence for involvement in exposure to stress, and is decreased in expression by omega-3 fatty acids in mouse brain. CCDC144B (Coiled-Coil Domain Containing 144B) was decreased in expression in blood in High Pain states. There is evidence in other tissues in human and animal model studies for involvement in pain. CCDC144B was a good predictor in the independent cohorts for state and trait, particularly for males with psychosis (SZ, SZA). It does not have trans-diagnostic evidence for involvement in other psychiatric disorders, seeming to be relatively specific for pain. GBP1 (Guanylate Binding Protein 1), with interferon induced signaling roles, is increased in expression in blood in High Pain states. There is other evidence in human studies, gene expression and genetic, for involvement in pain. GBP1 is a predictor in the independent cohorts for trait, particularly in females. It is increased in expression in the brain in MDD, schizophrenia, and suicide, and in blood in PTSD. GBP1 was decreased in expression by omega-3 in mouse brain. Hs.666804/MFAP3 (Microfibril Associated Protein 3), another of the top markers, is a component of elastin-associated microfibrils. MFAP3 had the most robust empirical evidence from the discovery and validation steps, and was a strong predictor in the independent cohort, particularly for pain in females and males with PTSD. Interestingly, it has no prior evidence for pain in the literature curated to date for the Prioritization/CFG step, which demonstrates that a wide-enough net was cast with the disclosed approach that can bring to the fore completely novel findings. MFAP3 was decreased in expression in blood in High Pain states, i.e., it is a pain suppressor gene. It also has previous evidence for involvement in alcoholism, stress, and suicide.

As disclosed herein, clustering analysis of a discovery cohort composed of participants with psychiatric disorders followed longitudinally over time, in which each participant had blood samples collected and neuropsychological testing done in at least one low pain state visit (Pain VAS ≤ 2 out of 10) and at least one high pain state visit (Pain VAS ≥6 out of 10), revealed two broad subtypes of high pain states: a predominantly psychotic subtype, possibly related to mis-connectivity and increased perception of pain centrally, and a predominantly anxious subtype, possibly related to reactivity and increased physical health reasons for pain peripherally. The powerful longitudinal within-participant design was used to discover blood gene expression changes between self-reported low pain and high pain states. Some of these gene expression biomarkers were increased in expression in high pain states (being putative risk genes, or "algogenes"), and others were decreased in expression (being putative protective genes, or "pain suppressor genes").

Advantageously, the present disclosure enables precision medicine for pain, with objective diagnostics and targeted novel therapeutics. Given the massive negative impact of untreated pain on quality of life, the current lack of objective measures to determine appropriateness of treatment, and the severe addiction gateway potential of existing opioid-based pain medications, the present disclosure provides herein. The methods described herein provide objective biomarkers for pain, which is a subjective sensation. Further, the biomarkers provided herein are able to objectively determine pain state and predict future emergency department visits for pain, even more so when personalized by gender and diagnosis. The biomarkers are suitable for targeting using existing drugs and yielded new drug candidates.

In view of the above, it will be seen that the several advantages of the disclosure are achieved and other advantageous results attained. As various changes could be made in the above methods and systems without departing from the scope of the disclosure, it is intended that all matter contained in the above description and shown in the accompanying drawings shall be interpreted as illustrative and not in a limiting sense.

When introducing elements of the present disclosure or the various versions, embodiment(s) or aspects thereof, the articles "a", "an", "the" and "said" are intended to mean that there are one or more of the elements. The terms "comprising", "including" and "having" are intended to be inclusive and mean that there may be additional elements other than the listed elements.

**Table 1. Convergent Functional Evidence (CFE) for Top Candidate Biomarkers for Pain (n=60 genes, 65 probesets).**

| Gene Symbol/ Gene Name | Probesets | Step 1 | Step 2 External Converge nt Functiona l Genomics (CFG) Evidence ForInvolvement inPain | | Step 4 | Step 4 | Step 4 | Step 5 | Step 6 | CFE Polyevide nce Score for Involvem ent in Pain (Based on Steps 1-4) |
|---|---|---|---|---|---|---|---|---|---|---|
| | | Discovery in Blood | | Step 3 Validation in Blood | Best Significant Prediction of State- High Pain | Best Significant Prediction of Trait-Future ED visits for Pain in the first year (Cases/Total) | Best Significant Predictions of Trait- Future ED visits for Pain in all future years | Other Psychiatric and Related Disorders Evidence | | |
| | | (Directionof Change) Method/ Score/ % | | ANOVA p-value/ Score | (Cases/Total) ROC AUC/ p-value | | (Cases/Total) | | Drugs thatModulate the Biomarker in Opposite Direction to Pain | |
| | | | Score | | | ROC AUC/ p-value | OR/OR p-value | | | |
| | | Up to 6pts | Up to 12pts | Up to 6 pts | 8 All pts 6pts Gender 4pts Gender/Dx | 8 pts All 6pts Gender 4pts Gender/Dx | 8 pts All 6pts Gender 4pts Gender/Dx | | | |
| | | | | 6.81E-02/2 | | | **All** | | | |
| | | | | | | **Gender** | C:(239/501) | | | |
| | | | | | | Females | 1.28/1.03E-04** | | | |
| | | | | | **All** | C:(7/44) | L:(145/309) | | | |
| | | | | | C:(101/411) | 0.7/4.92E-02 | 1.22/1.70E-02 | | | |
| | | | | | 0.56/3.52E-02 | **Gender/Dx** | **Gender** | | | |
| | | | | | **Gender** | F-MDD | Females | | | |
| | | | | | Male | C:(4/11) 1/3.20E-02 | C: (13/47) | | | |
| **GNG7** G Protein Subunit Gamma 7 | 1566643_a_a t | (D) D E/4 59% | 6 | Stepwise | C:(85/346) | 0.82/4.45E-02 | 1.69/4.69E-02 | Alcohol BP Hallucinoge ns MDD Stress SZ | Omega-3 fatty acids | 34 |
| | | | | | 0.56/3.95E-02 | L:(2/6) | | | | |
| | | | | | | | Males | | | |
| | | | | | **Gender/Dx** | | C:(226/454) | | | |
| | | | | | M-SZ | F-PTSD | 1.28/**1.92E-04**** | | | |
| | | | | | C:(11/64) | C:(2/8) | L:(138/282) | | | |
| | | | | | 0.68/2.79E-02 | 0.92/4.78E-02 | 1.21/2.16E-02 | | | |
| | | | | | | | **Gender/Dx** | | | |
| | | | | | | | F-MDD | | | |
| | | | | | | | C: (4/12) 14.54/2.23E-02 | | | |
| | | | | | | | M-MDD L:(25/43) 1.8/2.70E-02 | | | |
| | | | | | | | M-PSYCHOSIS C:(95/201) 1.52/**1.70E-04**** | | | |
| | | | | | | | L:(57/120) 1.34/2.47E-02 | | | |
| | | | | | | | M-SZ C:(42/103) 1.58/2.08E-02 | | | |
| | | | | | | | M-SZA C:(53/98) 1.71/**4.40E-04**** | | | |
| **CNTN1** Contactin 1 | 1554784_at | (D) D E/4 52% | 6 | NS | **All** C:(101/411) 0.58/1.15E-02 | **Gender** Males C:(95/426) 0.56/3.08E-02 | **Gender/Dx** M-MDD C:(42/72) 1.44/1.23E-02 | BP MDD SZ Suicide | Clozapine | 28 |
| | | | | | L:(61/248) 0.63/1.42E-03 | | | | | |
| | | | | | **Gender** Female C:(16/65) 0.65/3.38E-02 Male | | | | | |
| | | | | | L:(51/212) 0.63/2.27E-03 | | | | | |
| | | | | | | | L:(25/43) 1.64/4.17E-02 | | | |
| | | | | | **Gender/Dx** M-BP C:(24/123) 0.61/4.13E-02 | | | | | |
| | | | | | L:(16/81) 0.64/4.06E-02 M-SZ | | | | | |
| | | | | | C:(11/64) 0.68/3.15E-02 M-MDD | | | | | |
| | | | | | L:(13/43) 0.66/4.53E-02 M-SZA | | | | | |
| | | | | | L:(3/17) 0.83/3.89E-02 | | | | | |
| | | | | | **All** C:(101/411) 0.56/4.40E-02 | **All** C:(102/470) 0.56/2.30E-02 | **Gender/Dx** M-MDD C:(42/72) 1.65/3.85E-03 | | | |
| | | | | | L:(61/248) 0.58/2.39E-02 | | | | | |
| | | | | | **Gender** Male C:(85/346) 0.57/3.02E-02 | | | | | |
| | | | | | | **Gender** Males C:(95/426) 0.59/2.61E-03 | | | | |
| **LY9** Lymphocyte Antigen 9 | 231124_x_at | (I) DE/6 90% | 2 | NS | L:(51/212) 0.62/5.19E-03 | | | Acute Stress | Omega-3 fatty acids | 28 |
| | | | | | **Gender/Dx** M-BP C:(24/123) 0.63/2.66E-02 | **Gender/Dx** M-BP C:(18/120) 0.68/6.91E-03 | L:(25/43) 1.53/3.74E-02 | | | |
| | | | | | | | M-PTSD L:(18/20) 2.07/6.77E-03 | | | |
| | | | | | F-MDD C:(2/18) 0.97/1.75E-02 | M-PTSD L:(10/16) 0.77/4.13E-02 | | | | |
| | | | | | M-MDD L:(13/43) 0.8/9.87E-04 | | | | | |
| **CCDC144B** Coiled-Coil Domain Containing 144B (Pseudogene) | 1557366_at | (D) DE/4 56% | 6 | NS | **Gender/Dx** F-BP C:(4/21) 0.79/3.66E-02 | **Gender/Dx** M-MDD | **All** C:(239/501) 1.23/2.27E-03 | | | 26 |
| | | | | | M-PSYCHOSIS C:(19/96) 0.68/8.95E-03 | C:(26/67) 0.63/3.43E-02 | **Gender** Males C:(226/454) 1.23/3.34E-03 | | | |
| | | | | | L:(10/56) 0.68/4.16E-02 | | | | | |
| | | | | | M-SZA L:(3/17) 0.9/1.61E-02 | | **Gender/Dx** M-PSYCHOSIS C:(95/201) 1.41/3.46E-03 | | | |
| | | | | | | | L:(57/120) 1.43/1.32E-02 | | | |
| | | | | | | | M-SZ C:(42/103) 1.84/4.65E-03 | | | |
| | | | | | | | M-SZA L:(32/56) 1.47/3.49E-02 | | | |
| **GBP1** Guanylate Binding Protein 1 | 231578_at | (I) DE/2 37% | 6 | 3.26E-01/2 Stepwise | | **All** C:(102/470) 0.59/3.51E-03 | **All** C:(239/501) 1.09/3.72E-02 | MDD PTSD SZ | Omega-3 fatty acids | 26 |
| | | | | | | **Gender** Females | | | | |
| | | | | | | C:(7/44) 0.71/4.30E-02 | **Gender** Females C:(13/47) 1.68/2.41E-02 | | | |
| | | | | | | Males C:(95/426) 0.58/1.04E-02 | | | | |
| | | | | | | | **Gender/Dx** F-MDD C:(4/12) 3.1/4.43E-02 | | | |
| | | | | | | **Gender/Dx** F-MDD C:(4/11) 0.93/1.17E-02 | | | | |
| | | | | | | | M-SZA C:(53/98) 1.22/3.65E-02 | | | |
| | | | | | | M-PSYCHOSIS C:(33/198) 0.6/3.25E-02 M-SZA | | | | |
| | | | | | | C:(23/97) 0.62/4.10E-02 | | | | |
| **Hs.666804/ MFAP3** Microfibril Associated Protein 3 | 240949_x_at | (D) DE/6 81% | 0 | 6.03E-04/4 Nominal | **Gender/Dx** F-PTSD C:(5/12) 0.8/4.41E-02 | **Gender/Dx** M-BP L:(9/80) 0.75/7.27E-03 | **All** L:(145/309) 1.28/2.28E-02 | Alcohol Suicide Stress | | 26 |
| | | | | | | | **Gender** Males C:(226/454) 1.17/2.64E-02 L:(138/282) 1.35/8.94E-03 | | | |
| | | | | | | | **Gender/Dx** M-BP L:(34/91) 2.36/**4.86E-04**** | | | |
| | | | | | | | M-PTSD L:(18/20) 15.93/8.46E-04 | | | |
| **CASP6** Caspase 6 | 209790_s_at | (I) D E/4 51% | 4 | NS | **Gender** Male L:(51/212) 0.59/2.92E-02 | **Gender** Males C:(95/426) 0.57/2.54E-02 | **Gender/Dx** M-MDD C:(42/72) 1.31/3.97E-02 | BP | | 24 |
| | | | | | **Gender/Dx** F-MDD C:(2/18) 1/1.23E-02 | | | | | |
| | | | | | | **Gender/Dx** M-PSYCHOSIS C:(33/198) 0.6/2.88E-02 M-SZA C:(23/97) 0.63/2.71E-02 | | | | |
| | | | | | M-MDD L:(13/43) 0.87**/7.01E-05**** | | | | | |
| **COMT** Catechol-O-Methyltransferase | 216204_at | (D) D E/4 54% | 4 | NS | **Gender/Dx** M-MDD L:(13/43) 0.71/1.41E-02 | **All** C:(102/470) 0.55/4.48E-02 | **Gender/Dx** M-BP L:(34/91) 1.65/2.20E-02 | ADHD Aggression Alcohol Anxiety BP | Clozapine Morphine Mood Stabilizers | 24 |
| | | | | | | **Gender** Males | | | | |
| | | | | | | C:(95/426) 0.57/1.95E-02 | | Chronic Stress MDD OCD Panic Disorder Psychosis PTSD Suicide SZ | | |
| | | | | | | Gender/Dx M-MDD C:(26/67) 0.66/1.58E-02 M-PSYCHOSIS C:(33/198) 0.6/3.63E-02 | | | | |
| **RAB33A** RAB33A, Member RAS Oncogene Family | 206039_at | (I) DE/6 90% | 0 | NS | **Gender/Dx** F-MDD C:(2/18) 1/1.23E-02 | **Gender** Males C:(95/426) 0.56/3.60E-02 | **All** C:(239/501) 1.14/2.21E-02 | Alcohol Stress MDD | | 24 |
| | | | | | | | **Gender** Males C:(226/454) 1.16/1.01E-02 | | | |
| | | | | | | | **Gender/Dx** M-BP L:(34/91) 1.65/1.69E-03 | | | |
| | | | | | | | M-MDD C:(42/72) **1.95/6.59E-04**** | | | |
| | | | | | | | L:(25/43) 1.85/1.72E-02 | | | |
| **ZYX** Zyxin | 238016_s_at | (D) D E/4 57% | 4 | NS | **Gender/Dx** F-BP C:(4/21) 0.78/4.44E-02 | **All** C:(102/470) 0.55/4.80E-02 | **Gender/Dx** M-BP L:(34/91) 1.85/1.67E-02 | MDD | Clozapine | 24 |
| | | | | | | **Gender** Males C:(95/426) 0.57/1.58E-02 | | | | |
| | | | | | | | M-PTSD C:(26/31) 1.57/4.40E-02 | | | |
| | | | | | | | L:(18/20) 2.2/1.53E-02 | | | |
| | | | | | | **Gender/Dx** M-PSYCHOSIS | | | | |
| | | | | | | M-SZA C:(23/97) 0.66/1.15E-02 | | | | |
| | | | | | | M-BP L: (9/80) 0.71/2.26E-02 | | | | |
| **(Hs.696420) MTERF1** Mitochondrial Transcription Termination Factor 1 | 243125_x_at | (D) DE/6 100% | 0 | NS | | **Gender/Dx** F-PTSD C:(2/8) 1/2.28E-02 | **All** C:(239/501) 1.19/1.19E-02 L:(145/309) 1.2/4.81E-02 | PTSD Suicide | | 22 |
| | | | | | **Gender/Dx** M-PSYCHOSIS C:(19/96) 0.67/1.01E-02 | | **Gender** Males C:(226/454) 1.19/1.51E-02 | | | |
| | | | | | M-SZ C:(11/64) 0.77/2.27E-03 | | **Gender/Dx** M-PSYCHOSIS C:(95/201) 1.41/8.86E-03 | | | |
| | | | | | L: (7/39) 0.71/3.95E-02 | | | | | |
| | | | | | | | M-SZ C:(42/103) 1.4/4.47E-02 | | | |
| | | | | | | | M-SZA C:(53/98) 1.44/4.72E-02 | | | |
| **COL27A1** Collagen Type XXVII Alpha 1 Chain | 225293_at | (D) DE/4 79% | 4 | 7.47E-01/2 Stepwise | **Gender/Dx** M-MDD L:(13/43) 0.66/4.79E-02 | **Gender/Dx** M-MDD C:(26/67) 0.63/3.38E-02 | **Gender/Dx** M-PTSD L:(18/20) 1.96/2.37E-02 | Tourette syndrome | Lithium | 22 |
| | | | | | | M-PSYCHOSIS C:(33/198) 0.61/2.79E-02 | | | | |
| | | | | | | M-SZA C:(23/97) 0.68/4.96E-03 | | | | |
| | | | | | | L:(13/55) 0.7/1.62E-02 | | | | |
| **HRAS** HRas Proto-Oncogene, GTPase | 212983_at | (I) DE/6 97% | 0 | NS | **All** C:(101/411) 0.56/3.47E-02 | **Gender/Dx** F-PTSD C:(2/8) 1/2.28E-02 | **Gender/Dx** M-MDD C:(42/72) 2.2/**3.38E-06**** | Alcohol BP Longevity Suicide SZ | ISIS 2503 | |
| | | | | | L:(61/248) 0.58/3.01E-02 | | | | | |
| | | | | | **Gender** Male C:(85/346) 0.57/2.72E-02 | | | | | 22 |
| | | | | | L:(51/212) 0.61/1.18E-02 | | | | | |
| | | | | | | | L:(25/43) 2.25/**2.61E-04**** | | | |
| | | | | | **Gender/Dx** M-SZ C:(11/64) 0.68/2.79E-02 | | | | | |
| | | | | | M-MDD L:(13/43) 0.71/1.61E-02 | | | | | |
| **CALCA** Calcitonin Related Polypeptide Alpha | 210727_at | (D) DE/4 54% | 7 | NS | **Gender** Females C:(16/63) 0.66/3.12E-02 | **Gender/Dx** F-PTSD C:(2/8) 1/2.28E-02 Gender/Dx M-PSYCHOSIS C:(33/198) 0.6/3.87E-02 | | Alcohol Anxiety Panic Disorder | Omega-3 fatty acids Lithium | 21 |
| | | | | | Gender/Dx F-MDD C:(2/18) 0.97/1.75E-02 | | | | | |
| | | | | | F-BP L:(3/11) 0.88/3.31E-02 | | | | | |
| | | | | | M-MDD L:(13/43) 0.66/4.79E-02 | | | | | |
| **(Hs.596713) PPP1R14B** Protein Phosphatase 1 Regulatory Inhibitor Subunit 14B | 226138_s_at | (D) DE/6 90% | 0 | 6.28E-02/2 Stepwise | **Gender/Dx** F-BP C:(4/21) 0.94/3.61E-03 | | **All** C:(239/501) 1.15/1.43E-02 | SZ | Lithium | 20 |
| | | | | | | | **Gender** Males C:(226/454) 1.19/4.84E-03 | | | |
| | | | | | | | L:(138/282) 1.2/3.94E-02 | | | |
| | | | | | L:(3/11) 0.92/2.06E-02 M-MDD | | **Gender/Dx** M-PSYCHOSIS C:(95/201) 1.35/3.06E-03 | | | |
| | | | | | L:(13/43) 0.73/9.98E-03 | | M-SZ C:(42/103) 1.53/3.19E-02 | | | |
| | | | | | | | M-SZA C:(53/98) 1.41/9.26E-03 | | | |
| **ASTN2** Astrotactin 2 | 1554816_at | (I) DE/6 83% | 2 | 1.71E-01 /2 Stepwise | | **Gender/Dx** F-MDD L:(2/6) 1/3.20E-02 | **Gender** Female L:(7/27) 2.45/4.36E-02 | Suicide SZ ASD BP MDD | Antipsychoti cs | 20 |
| **ELAC2** ElaC Ribonuclease Z 2 | 201766_at | (D) DE/4 52% | 2 | 4.11E-02/4 Nominal | **Gender/Dx** M-MDD | | **Gender** Males L:(138/282) 1.2/4.61E-02 | ASD | | 20 |
| | | | | | L:(13/43) 0.73/8.66E-03 | | **Gender/Dx** M-BP L:(34/91) 1.55/4.79E-02 | | | |
| | | | | | | | M-MDD C:(42/72) 1.69/2.47E-03 | | | |
| | | | | | | | L:(25/43) 1.85/3.66E-02 | | | |
| **HLA-DQB1** Major Histocompatibility Complex, Class II, DQ Beta 1 | 212998_x_at | (I) D E/4 51% | 8 | NS | **Gender/Dx** M-SZ C:(11/64) 0.68/3.41E-02 | | **Gender/Dx** M-BP L:(34/91) 1.63/1.30E-02 | Alcohol Depression Longevity Stress Suicide SZ | Antipsychoti cs | 20 |
| | | | | | F-MDD C:(2/18) 1/1.23E-02 | | | | | |
| | | | | | M-MDD L:(13/43) 0.67/4.28E-02 | | | | | |
| **HLA-DQB1** Major Histocompatibility Complex, Class II, DQ Beta 1 | 211656_x_at | (I) D E/4 59% | 8 | NS | **Gender/Dx** F-MDD C:(2/18) 1/1.23E-02 | **Gender/Dx** M-MDD C:(26/67) 0.62/4.85E-02 | | Alcohol BP Depression Longevity PTSD Stress Suicide SZ | Antipsychoti cs | 20 |
| | | | | | M-SZ C:(11/64) 0.68/3.15E-02 | | | | | |
| | | | | | M-SZ C:(11/64) 0.74/5.90E-03 L:(7/39) 0.72/3.36E-02 | | | | | |
| | | | | | M-MDD | | | | | |
| | | | | | L:(13/43) 0.69/2.68E-02 | | | | | |
| | | | | | M-PSYCHOSIS L:(10/56) 0.69/3.29E-02 | | | | | |
| **PNOC** Prepronociceptin | 205901_at | (I) DE/4 62% | 4 | NS | **Gender/Dx** M-SZ L:(7/39) 0.72/3.36E-02 | **Gender/Dx** M-BP L: (9/80) 0.68/4.20E-02 | **Gender/Dx** M-BP C:(53/134) 1.23/4.73E-02 | Addictions BP MDD SZ Stress | | 20 |
| | | | | | | | L:(34/91) 1.26/2.67E-02 | | | |
| | | | | | | | M-MDD C:(42/72) 1.4/2.09E-02 | | | |
| **TCF15** Transcription Factor 15 (Basic Helix-Loop-Helix) | 207306_at | (D) DE/6 94% | 2 | NS | | | **All** C:(239/501) 1.11/4.85E-02 | Suicide | | 20 |
| | | | | | **Gender/Dx** F-MDD C:(2/18) 0.94/2.46E-02 | | **Gender** Males C:(226/454) 1.14/2.39E-02 | | | |
| | | | | | M-MDD L:(13/43) 0.68/3.21E-02 | | **Gender/Dx** M-BP L:(34/91) 2.22/2.61E-03 | | | |
| | | | | | | | **All** L:(145/309) 1.18/4.66E-02 | | | |
| **TOP3A** Topoisomerase (DNA) III Alpha | 214300_s_at | (D) DE/4 51% | 4 | NS | **Gender/Dx** F-BP C:(4/21) 0.84/1.97E-02 | | **Gender** Males L:(138/282) 1.2/3.88E-02 | | Omega-3 fatty acids | 20 |
| | | | | | | | **Gender/Dx** M-SZ L:(25/64) 1.75/4.72E-02 | | | |
| | | | | | | **All** C:(102/470) 0.56/2.27E-02 L:(58/287) 0.58/3.38E-02 | | | | |
| | | | | | | **Gender** Males C:(95/426) 0.57/1.64E-02 | | | | |
| | | | | | | L:(54/261) 0.59/2.71E-02 | | | | |
| **(H05785)** LRRC75A Leucine Rich Repeat Containing 75A | 236913_at | (D) AP/6 97% | 0 | NS | **Gender/Dx** F-MDD C:(2/18) 0.94/2.46E-02 | **Gender/Dx** F-PTSD C:(2/8) 1/2.28E-02 M-PSYCHOSIS | | Alcohol BP Suicide SZ | Clozapine | 18 |
| | | | | | | C:(33/198) 0.65/3.29E-03 M-SZA | | | | |
| | | | | | | C:(23/97) 0.68/5.21E-03 M-SZA | | | | |
| | | | | | | L:(13/55) 0.66/4.42E-02 M-MDD | | | | |
| | | | | | | L:(16/39) 0.76/3.64E-03 | | | | |
| **CLSPN** Claspin | 242150_at | (I) AP/6 95% | 0 | NS | **Gender/Dx** M-PSYCHOSIS C:(19/96) 0.65/2.48E-02 | **All** L: (58/287) 0.57/4.62E-02 | | Suicide | | 18 |
| | | | | | | **Gender/Dx** F-MDD L:(2/6) 1/3.20E-02 | | | | |
| | | | | | | M-MDD L:(16/39) 0.67/4.08E-02 | | | | |
| **COL2A1** Collagen Type II Alpha 1 Chain | 217404_s_at | (D) D E/4 54% | 4 | NS | | **Gender** Males C:(95/426) 0.56/3.53E-02 | **Gender/Dx** M-PTSD C:(26/31) 1.83/4.38E-03 | Aging | | 18 |
| | | | | | | **Gender/Dx** M-PSYCHOSIS C:(33/198) 0.63/7.32E-03 | | | | |
| | | | | | | | L:(18/20) 2.3/1.08E-02 | | | |
| | | | | | | M-SZA C:(23/97) 0.66/1.08E-02 | | | | |
| | | | | | | L:(13/55) 0.66/3.73E-02 | | | | |
| **HLA-DQB1** Major Histocompatibility Complex, Class II, DQ Beta 1 | 210747_at | (D) DE/2 44% | 8 | NS | | | **All** C:(239/501) 1.17/1.03E-02 | Addiction Stress | Benzodiazepi nes | 18 |
| | | | | | | | **Gender** Males C:(226/454) 1.19/6.06E-03 | | | |
| | | | | | | | **Gender/Dx** M-MDD C:(42/72) 1.35/3.68E-02 | | | |
| | | | | | | | M-PSYCHOSIS C:(95/201) 1.26/1.33E-02 | | | |
| | | | | | | | M-SZA C:(53/98) 1.33/2.06E-02 | | | |
| | | | | | | **All** C:(102/470) 0.56/2.38E-02 | **Gender/Dx** F-MDD C:(4/12) 7/4.47E-02 | | | |
| | | | | | | L:(58/287) 0.58/2.49E-02 | | | | |
| | | | | | | **Gender** Males C:(95/426) 0.56/4.18E-02 | | | | |
| Hs.554262 | 210703_at | (I) AP/6 100% | 0 | NS | | L:(54/261) 0.59/1.65E-02 | | Suicide | | 18 |
| | | | | | | **Gender/Dx** F-MDD C:(4/11) 0.82/4.45E-02 | M-MDD L:(25/43) 2.13/7.30E-03 | | | |
| | | | | | | M-BP C:(18/120) 0.67/1.08E-02 | | | | |
| | | | | | | M-MDD L:(16/39) 0.67/4.08E-02 | | | | |
| **PIK3CD** Phosphatidylinositol-4,5-Bisphosphate 3-Kinase Catalytic Subunit Delta | 211230_s_at | (D) DE/6 83% | 0 | 1.59E-02/4 Nominal | | | **All** C:(239/501) 1.13/3.18E-02 | Alcohol Chronic Stress Longevity Suicide SZ | Clozapine Lithium Valproate | 18 |
| | | | | | | | **Gender** Males C:(226/454) 1.14/2.71E-02 | | | |
| | | | | | | | **Gender/Dx** M-BP C:(53/134) 1.3/2.85E-02 | | | |
| | | | | | | | L:(34/91) 1.57/2.01E-02 | | | |
| | | | | | | | M-MDD C:(42/72) 1.65/5.12E-03 | | | |
| **SVEP1** Sushi, Von Willebrand Factor Type A, EGF And Pentraxin Domain Containing 1 | 236927_at | (I) DE/2 49% | 4 | 2.17E-02/4 Nominal | **Gender/Dx** F-PTSD C:(5/12) 0.8/4.41E-02 | **Gender/Dx** F-MDD C:(4/11) 0.82/4.41E-02 | | Addiction SZ | Omega-3 fatty acids | 18 |
| | | | | | M-PTSD C:(13/38) 0.67/4.68E-02 | | | | | |
| **TNFRSF11B** TNF Receptor Superfamily Member 11b | 204932_at | (D) DE/2 37% | 4 | 2.67E-02/4 Nominal | **Gender/Dx** F-BP C:(4/21) 0.81/3.00E-02 M-MDD | | **Gender/Dx** M-MDD C:(42/72) 1.42/4.25E-02 | Stress PTSD | | 18 |
| | | | | | L:(13/43) 0.71/1.72E-02 | 13/43) | L:(25/43) 1.59/3.84E-02 | | | |
| **ZNF91** Zinc Finger Protein 91 | 244259_s_at | (I) AP/6 95% | 0 | 6.37E-01/2 Stepwise | | **Gender/Dx** F-MDD C:(4/11) 0.93/1.17E-02 | **Gender** Females C:(13/47) 2.12/1.03E-02 | Alcohol Circadian abnormalitie s PTSD | | 18 |
| | | | | | | | **Gender/Dx** F-BP C:(2/16) 4.21/4.55E-02 | | | |
| | | | | | | | M-BP C:(53/134) 1.35/1.26E-02 | | | |
| **CDK6** Cyclin Dependent Kinase 6 | 224851_at | (I) DE/4 56% (I) AP/2 42% | 4 | NS | **Gender/Dx** F-BP | **All** C:(102/470) 0.57/1.03E-02 | | Alcohol ASD Circadian abnormalitie s Longevity MDD SZ | | 17 |
| | | | | | C:(4/21) 0.78/4.44E-02 | **Gender** Males C:(95/426) 0.59/5.57E-03 | | | | |
| | | | | | L:(3/11) 1/7.15E-03 | | | | | |
| | | | | | | **Gender/Dx** M-MDD C:(26/67) 0.67/9.11E-03 | | | | |
| **EDN1** Endothelin 1 | 1564630_at | (I) AP/4 56% | 4 | 8.69E-02/2 Stepwise | | | **Gender** Females C:(13/47) 1.9/1.48E-02 | | | 16 |
| | | | | | | | **Gender/Dx** M-BP C:(53/134) 1.27/2.37E-02 | | | |
| **(AF090920) PPFIBP2** PPFIA Binding Protein 2 | 234739_at | (I) AP/6 94% | 0 | NS | **Gender** Female C:(16/65) 0.68/1.42E-02 | | **Gender/Dx** M-PSYCHOSIS C:(95/201) 1.19/3.77E-02 | | | 16 |
| | | | | | L:(10/36) 0.69/3.87E-02 | | | | | |
| | | | | | **Gender/Dx** F-PTSD C:(5/12) 0.8/4.41E-02 | | M-SZ C:(42/103) 1.22/4.66E-02 | | | |
| **DCAF12** DDB1 And CUL4 Associated Factor 12 | 224789_at | (D) DE/6 86% | 2 | NS | **Gender/Dx** F-MDD C:(2/18) 1/1.23E-02 | | **Gender/Dx** M-BP C:(53/134) 1.61/4.42E-03 | Cocaine Suicide | Omega-3 fatty acids Clozapine | 16 |
| **DNAJC18** DnaJ Heat Shock Protein Family (Hsp40) Member C18 | 227166_at | (I) DE/6 94% | 0 | NS | **Gender** Female L:(10/36) 0.78/4.97E-03 | | | BP | | 16 |
| | | | | | **Gender/Dx** F-SZA L: (3/8) 0.93/2.63E-02 | **Gender/Dx** F-MDD | | | | |
| | | | | | F-BP L:(3/11) 0.88/3.31E-02 | C:(4/11) 0.93/1.17E-02 | | | | |
| | | | | | F-PSYCHOSIS L: (3/8) 0.93/2.63E-02 | | | | | |
| | | | | | F-PTSD L:(3/6) 1/2.48E-02 | | | | | |
| **HLA-DRB1** Major Histocompatibility Complex, Class II, DR Beta 1 | 208306_x_at | (I) AP/4 52% | 4 | NS | **Gender/Dx** F-MDD C:(2/18) 0.91/3.39E-02 M-MDD | **Gender/Dx** M-SZA C:(23/97) 0.62/4.69E-02 | | Stress PTSD | Antipsychoti cs | 16 |
| | | | | | L:(13/43) 0.66/4.79E-02 M-SZ L:(7/39) 0.71/4.27E-02 | | | | | |
| **SEPT7P2** Septin 7 Pseudogene 2 | 1569973_at | **(I) DE/6 100%** (I) AP/2 39% | 0 | NS | **Gender** Females C:(16/65) 0.65/3.27E-02 | | **Gender/Dx** M-MDD C:(42/72) 1.45/1.37E-02 | Suicide | | 16 |
| | | | | | | | L:(25/43) 2.25/**5.24E-04**** | | | |
| | | | | | **Gender/Dx** F-PTSD C:(5/12) 0.97/3.69E-03 | | M-PTSD C:(26/31) 2.38/**7.38E-04**** | | | |
| | | | | | M-SZ C:(11/64) 0.77/2.83E-03 | | L:(18/20) 3.59/1.77E-03 | | | |
| | | | | | | | | | | |
| **VEGFA** Vascular Endothelial Growth Factor A | 212171_x_at | (I) AP/4 65% | 4 | NS | **Gender/Dx** M-PSYCHOSIS C:(19/96) 0.66/1.78E-02 | | **Gender/Dx** M-MDD C:(42/72) 1.33/4.83E-02 | BP MDD Stress SZ | Lithium Valproate Olanzapine | 16 |
| | | | | | M-SZA C:(8/32) 0.7/4.48E-02 | | | | | |
| **WNK1** WNK Lysine Deficient Protein Kinase 1 | 1555068_at | (D) DE/6 92% | 2 | NS | **Gender/Dx** M-MDD L:(13/43) 0.77/2.75E-03 | | **Gender/Dx** M-BP C:(53/134) 1.41/3.18E-02 | Alcohol Depression Suicide Methamphet amine Stress | Omega-3 Fatty acids SSRI | 16 |
| **(AF087971) PBRM1** Polybromo 1 | 1561067_at | (I) AP/6 90% | 0 | NS | | **All** C:(102/470) 0.56/3.71E-02 | | BP Hallucinatio ns Longevity MDD Methamphet amine Mood | | 14 |
| | | | | | | **Gender** Males C:(95/426) | | | | |
| | | | | | | 0.56/2.87E-02 | | Psychosis Stress Suicide | | |
| | | | | | | **Gender/Dx** M-BP C:(18/120) 0.63/3.95E-02 M-PSYCHOSIS | | | | |
| | | | | | | C:(33/198) 0.63/8.63E-03 M-SZA | | | | |
| | | | | | | C:(23/97) 0.66/1.26E-02 | | | | |
| **(Hs.609761) SFPQ** Splicing Factor Proline And Glutamine Rich | 244331_at | (D) DE/6 98% | 0 | NS | **Gender/Dx** M-SZ C:(11/64) 0.68/3.28E-02 | | **Gender/Dx** M-MDD C:(42/72) 1.68/7.35E-03 | Alcohol BP MDD Stress Suicide | Omega-3 fatty acids Clozapine Antidepressa nts Antipsychoti cs | 14 |
| | | | | | L:(7/39) 0.75/2.21E-02 | | | | | |
| **(Hs.659426) PHC3** Polyhomeotic Homolog 3 | 240599_x_at | (D) DE/6 92% | 0 | NS | **Gender/Dx** F-MDD C:(2/18) 0.91/3.39E-02 | | Gender/Dx M-MDD C:(42/72) 1.48/1.83E-02 | Suicide | | 14 |
| **CCDC85C** Coiled-Coil Domain Containing 85C | 219018_s_at | (D) DE/6 94% | 2 | NS | **Gender** Female L:(10/36) 0.7/3.31E-02 | | | Suicide | | 14 |
| | | | | | **Gender/Dx** F-BP C:(4/21) 0.79/3.66E-02 | | | | | |
| | | | | | L:(3/11) 0.92/2.06E-02 | | | | | |
| | | | | | F-PTSD L:(3/6) 1/2.48E-02 | | | | | |
| **GSPT1** G1 To S Phase Transition 1 | 215438_x_at | (D) DE/6 94% | 0 | NS | **Gender/Dx** F-MDD C:(2/18) 1/1.23E-02 | | **Gender/Dx** M-BP C:(53/134) 1.58/4.92E-03 | BP Suicide MDD | Valproate | 14 |
| **HLA-DQB1** Major Histocompatibility Complex, Class II, DQ Beta 1 | 211654_x_at | (I) DE/2 40% | 8 | NS | **Gender/Dx** M-PSYCHOSIS L:(10/56) 0.73/1.23E-02 | | | Alcohol BP Depression Longevity PTSD Stress Suicide SZ | Antipsychoti cs | 14 |
| | | | | | M-SZ L:(7/39) 0.81/5.78E-03 | | | | | |
| **LOXL2** Lysyl Oxidase Like 2 | 228808_s_at | (D) D E/4 59% | 4 | NS | **Gender** Females C:(16/65) 0.66/3.05E-02 | | | BP Suicide | | 14 |
| | | | | | **Gender/Dx** F-MDD C:(2/18) 1/1.23E-02 | | | | | |
| **MBNL3** Muscleblind Like Splicing Regulator 3 | 219814_at | (D) DE/6 92% | 0 | NS | **Gender/Dx** M-MDD L:(13/43) 0.71/1.51E-02 | | **Gender/Dx** M-BP C:(53/134) 1.43/8.16E-03 | Psychosis Hallucinatio n | | 14 |
| **PTN** Pleiotrophin | 211737_x_at | (D) DE/6 92% | 0 | NS | | | **All** C:(239/501) 1.16/1.17E-02 | SZ Stress Suicide | Omega-3 fatty acids Risperidone | 14 |
| | | | | | | | **Gender** Males | | | |
| | | | | | | | C:(226/454) 1.2/4.66E-03 | | | |
| | | | | | | | **Gender/Dx** M-PSYCHOSIS C:(95/201) 1.24/1.98E-02 | | | |
| | | | | | | | M-SZA C:(53/98) 1.35/1.28E-02 | | | |
| **RALGAPA2** Ral GTPase Activating Protein Catalytic Alpha Subunit 2 | 231826_at | (D) DE/6 97% | 0 | NS | **Gender/Dx** F-MDD C:(2/18) 0.94/2.46E-02 | | **Gender/Dx** M-MDD C:(42/72) 2.06/**4.52E-04**** | BP | | 14 |
| | | | | | | | L:(25/43) 2.05/5.35E-03 | | | |
| **YBX3** Y-Box Binding Protein 3 | 201160_s_at | (D) DE/6 94% | 0 | NS | **Gender/Dx** F-MDD C:(2/18) 0.97/1.75E-02 | | **Gender/Dx** M-BP C:(53/134) 1.39/1.23E-02 | BP Suicide SZ | Mianserin | 14 |
| **ZNF441** Zinc Finger Protein 441 | 1553193_at | (I) AP/6 95% (I) DE/2 35% | 0 | NS | | **Gender/Dx** M-SZA L:(13/55) 0.67/3.13E-02 | **Gender/Dx** M-MDD L:(25/43) 1.72/1.92E-02 | | | 14 |
| **CCND1** Cyclin D1 | 208712_at | (D) DE/4 57% | 4 | NS | | | **Gender/Dx** M-BP C:(53/134) 1.33/4.53E-02 | Addiction MDD Stress Hallucinoge ns | | 12 |
| **CDK6** Cyclin Dependent Kinase 6 | 224847_at | (I) DE/4 63% | 4 | NS | | | **Gender/Dx** M-PTSD L:(18/20) 2.09/1.75E-02 | Alcohol ASD Circadian abnormalitie s Longevity MDD SZ | | 12 |
| **CO MT** Catechol-O-Methyltransferase | 213981_at | (D) DE/4 54% | 4 | NS | **Gender/Dx** M-MDD L:(13/43) 0.71/1.41E-02 | | | ADHD Aggression Alcohol Anxiety BP Chronic Stress MDD OCD Panic Disorder Psychosis PTSD Suicide SZ | Clozapine Morphine Mood Stabilizers | 12 |
| **HTR2A** 5-Hydroxytryptamine Receptor 2A | 211616_s_at | (D) DE/4 52% | 4 | NS | **Gender/Dx** M-BP L:(16/81) 0.65/2.89E-02 | | | Addictions Aging Alcohol Anxiety BP Depression MDD Mood Disorders NOS OCD Panic Disorder PTSD Stress Suicide SZ | | 12 |
| **NF1** Neurofibromin 1 | 212676_at | (I) DE/4 59% | 4 | NS | **Gender/Dx** F-BP L:(3/11) 0.92/2.06E-02 | | | Addiction BP PTSD | Fluoxetine SSRI | 12 |
| **SHMT1** Serine Hydroxymethyltransf erase 1 | 217304_at | (D) DE/2 43% | 6 | NS | | Gender/Dx F-PTSD C:(2/8) 1/2.28E-02 M-SZA | | Suicide | Clozapine | 12 |
| | | | | | | L:(13/55) 0.7/1.54E-02 | | | | |
| **TSPO** Translocator Protein | 202096_s_at | (I) DE/2 38% | 6 | NS | **Gender/Dx** M-SZ C:(11/64) 0.72/1.06E-02 | | | SZ | | 12 |
| **DENND1B** DENN Domain Containing 1B | 1557309_at | (I) DE/6 90%; (I) AP/2 40% | 0 | NS | **Gender/Dx** M-SZA L:(3/17) 0.83/3.89E-02 | | | | Omega-3 | 10 |
| **MCRS1** Microspherule Protein 1 | 202556_s_at | (I) DE/6 90% | 0 | NS | **Gender/Dx** M-MDD L:(13/43) 0.75/5.16E-03 | | | MDD | | 10 |
| **OSBP2** Oxysterol Binding Protein 2 | 1569617_at | (D) DE/6 94% | 0 | NS | **Gender/Dx** F-MDD C:(2/18) 1/1.23E-02 | | | Cocaine Suicide SZ | | 10 |
| **FAM134B** Family With Sequence Similarity 134 Member B | 218510_x_at | (I) DE/4 51%; (I) AP/2 34% | 4 | NS | | | | Antisocial Personality Suicide | Omega-3 Fatty acids | 8 |
| **ZNF429** Zinc Finger Protein 429 | 1561270_at | (D) DE/2 37% | 6 | NS | | | | | | 8 |
| **(Hs.677263) SMURF2** SMAD Specific E3 Ubiquitin Protein Ligase 2 | 216444_at | (D) AP/6 100% (D) DE/4 71% | 0 | NS | | | | Aging Suicide Stress | | 6 |

| | | | | | | | | | | |
|---|---|---|---|---|---|---|---|---|---|---|
| DE- differential expression, AP-Absent/Present. NS- Non-stepwise in validation. For Predictions, C-cross-sectional (using levels from one visit), L-longitudinal (using levels and slopes from multiple visits). In All, by Gender, and personalized by Gender and Diagnosis (Gender/Dx).M-males, F-Females. MDD-depression, BP-bipolar, SZ-schizophrenia, SZA-schizoaffective, PSYCHOSIS- schizophrenia and schizoaffective combined, PTSD-post-traumatic stress disorder. Bold and **-significant after Bonferroni correction for the number of biomarkers tested (65). For Steps 2, 5 and 6, see Supplementary Information tables for citations for the evidence. | | | | | | | | | | |

**Table 2. Therapeutics. New Drug Discovery/Repurposing.**

| **A. CMAP Top Biomarkers (n=65 probesets; 19 decreased, 14 increased are present in HG-U133A array used by CMAP)** | | | |
|---|---|---|---|
| rank | CMAP name | score | Description |
| 1 | **SC-560** | -1 | SC-560 is an NSAID, member of the diaryl heterocycle class of cyclooxygenase (COX) inhibitors which includes celecoxib (Celebrex^{™}) and rofecoxib (Vioxx^{™}). However, unlike these selective COX-2 inhibitors, SC-560 is a selective inhibitor of COX-1. |
| 2 | *pyridoxine* | -0.997 | Pyridoxine is the 4-methanol form of vitamin B6 and is converted to pyridoxal 5-phosphate in the body. Pyridoxal 5-phosphate is a coenzyme for synthesis of amino acids, neurotransmitters (serotonin, norepinephrine), sphingolipids, aminolevulinic acid. |
| 3 | methylergometrin e | - 0.975 | Methylergometrine is a synthetic analogue of ergonovine, a psychedelic alkaloid found in ergot, and many species of morning glory. It is chemically similar to LSD, ergine, ergometrine, and lysergic acid. Due to its oxytocic properties, it has a medical use in obstetrics. |
| 4 | LY-294002 | -0.923 | LY-294002 is a potent, cell permeable inhibitor of phosphatidylinositol 3-kinase (Pl3K) that acts on the ATP binding site of the enzyme. The PI3K pathway has a role in inhibiting apoptosis in cancer. PI3K is also known to regulate TLR-mediated inflammatory responses. |
| 5 | haloperidol | -0.917 | Widely used typical anti-psychotic medication |
| 6 | cytisine | -0.909 | Like varenicline, cytisine is a partial agonist of nicotinic acetylcholine receptors (nAChRs), with an affinity for the α4β2 receptor subtype, and a half-life of 4.8 hours. |
| 7 | *cyanocobalamin* | -0.902 | Cyanocobalamin is a form of vitamin B12. Vitamin B12 is important for growth, cell reproduction, blood formation, and protein and tissue synthesis. |
| 8 | *apigenin* | -0.899 | Apigenin (4',5,7-trihydroxyflavone), found in many plants such as chamomile, is a natural product belonging to the flavone class. Apigenin acts as a monoamine transporter activator, and is a weak ligand for central benzodiazepine receptors in vitro and exerts anxiolytic and slight sedative effects in an animal model. It has also effects on adenosine receptors and is an acute antagonist at the NMDA receptors (IC50 = 10 µM). In addition, like various other flavonoids, apigenin has been found to possess nanomolar affinity for the opioid receptors, acting as a non-selective antagonist of all three opioid receptors. |
| 9 | ***beta-escin*** | -0.892 | Escin, a natural mixture of triterpenoid saponins isolated from horse chestnut (*Aesculus hippocastanum*) seeds, is used and studied as a vasoprotective anti-inflammatory, anti-edematous and anti-nociceptive agent. |
| 13 | **amoxapine** | -0.875 | Amoxapine is a tricyclic antidepressant of the dibenzoxazepine class. This drug is used to treat symptoms of depression and neuropathic pain. |

| **B. L1000CDS2 Top Biomarkers (n=60 unique genes; 26 increased and 34 decreased).** | | | |
|---|---|---|---|
| **Rank** | **Score** | **Drug** | **Description** |
| 1 | 0.1458 | Quinethazone | Thiazide diuretic |
| 2 | 0.1458 | ***(-)-Gallocatechin gallate*** | Related to the green tea compound EGCG and possible therapeutic molecule for NP treatment due to its anti-inflammatory and antioxidant properties. Interestingly, it has been shown that EGCG reduced bone cancer pain. |
| 3 | 0.125 | ***EICOSATRIENOIC ACID (20:3 n-3)*** | Omega-3 fatty acid |
| 4 | 0.125 | LFM-A13 | Tyrosine kinase inhibitor with anti-inflamatory properties |
| 5 | 0.125 | Picrotoxinin | GABA and glycine receptors inhibitor |
| 6 | 0.125 | INDAPAMIDE | Thiazide-like diuretic |
| 7 | 0.125 | BRD-K15318909 | |
| 8 | 0.125 | BRD-K53011428 | |
| 9 | 0.125 | BRD-K35100517 | |
| 10 | 0.125 | MLS-0454435.0001 | |
| 11 | 0.125 | NCGC00181213-02 | |
| 12 | 0.125 | ST003833 | |
| 13 | 0.125 | STOCK2S-84516 | |
| 14 | 0.125 | MLS-0390932.0001 | |
| 15 | 0.125 | BRD-K98143437 | |
| 16 | 0.125 | BRD-A00993607 | |
| 17 | 0.125 | BRD-K68103045 | |
| 18 | 0.125 | BRD-K90700939 | |
| 19 | 0.125 | triamterene | potassium-sparing diuretic used in combination with thiazide diuretics for the treatment of hypertension and edema. |
| 20 | 0.1042 | PSEUDOEPHEDRINE HYDROCHLORIDE | sympathomimetic drug |
| 21 | 0.1042 | ***DOCOSAHEXAENOIC ACID (22:6 n-3)*** | Omega-3 fatty acid with antihyperalgesic effect in neuropathic pain |
| 22 | 0.1042 | *Evoxine* | Plant alkaloid with hypnotic and sedative effects. |
| 23 | 0.1042 | Gavestinel | NMDA receptor antagonist |
| 24 | 0.1042 | Mometasone furcate | Corticosteroid |
| 25 | 0.1042 | ZM 241385 | denosine A2A receptor antagonist |

| | | | |
|---|---|---|---|
| A. Connectivity Map (CMAP) analysis- drugs that have opposite gene expression profile effects to pain biomarkers signatures. Out of 65 probesets, 14 of the 29 increased, and 19 of the 36 decreased were present in HG-U133A array used by Connectivity Map. A score of -1 indicates the perfect opposite match, i.e., the best potential therapeutic for Pain. B. NIH LINCS analysis using the L1000CDS2 (LINCS L1000 Characteristic Direction Signature Search Engine) tool. Query for signature is done using gene symbols and direction of change. Shown are compounds mimicking direction of change in high memory. A higher score indicates a better match. Bold-drugs known to treat pain, which thus serve as a de facto positive control for the Example. Italic- natural compounds. | | | |

**Table 3. Demographics. MDD-depression, BP-bipolar, SZ-schizophrenia, SZA-schizoaffective, PSYCHOSIS- schizophrenia and schizoaffective combined, PTSD-post-traumatic stress disorder.**

| **Cohorts** | **Number of subjects** | **Gender** | **Diagnosis** | **Ethnicity** | **Age at time of visit Mean (SD)** | **T-test for age** |
|---|---|---|---|---|---|---|
| **Discovery** | | | | | | |
| Discovery Cohort (Longitudinal Within-Subject Changes in Pain Scale) Low Pain 0-2 to High Pain 6-10 | 28 (with 79 visits) | Male = 19 | BP = 9 | EA= 17 | 52 (7.94) | |
| | | | MDD= 3 | | | |
| | | | SZA= 6 | | | |
| | | Female = 9 | SZ= 3 | AA= 10 | | |
| | | | PTSD= 5 | Mixed = 1 | | |
| | | | PSYCH= 2 | | | |

| **Validation** | | | | | | |
|---|---|---|---|---|---|---|
| Independent Validation Cohort (Clinical Severe Pain Diagnosis SF36 sum of scores on questions 21 and 22 ≥10 Pain Scale ≥6) | 23 (30 visits) | Male = 13 | MDD=8 | | 51.9 (7.1) | |
| | | | BP=6 | | | |
| | | | SZ=2 | EA= 17 | | |
| | | Female = 10 | SZA=2 | AA= 6 | | |
| | | | PTSD=2 | | | |
| | | | MOOD=3 | | | |

| **Testing** | | | | | | |
|---|---|---|---|---|---|---|
| Independent Testing Cohort For Predicting State (High Pain State Pain Scale ≥6 at Time of Assessment) | 162 (411 visits) | Male = 134 | BP=52 | EA= 112 | 50.3 (8.97) Others 50.12 High Pain 50.50 | High Pain (n=101) Vs. Others (n=310) **0.824** |
| | | | MDD=39 | | | |
| | | | SZA=19 | | | |
| | | Female = 28 | SZ=26 | AA= 48 | | |
| | | | PTSD=20 | Hispanic=2 | | |
| | | | MOOD=4 | | | |
| | | | PSYCH=2 | | | |
| Independent Testing Cohort For Predicting Trait (Future ED visits for Pain in the First Year Following Assessment) | 181 (470 visits) | Male = 163 | BP = 46 | EA= 117 | 52.45 (6.13) Others 52.61 ED visits for Pain 51.87 | ED visits for Pain (n=102) vs. Others (n=368) **0.237** |
| | | | MDD= 33 | | | |
| | | | SZA= 45 | | | |
| | | Female = 18 | SZ= 38 | AA= 62 | | |
| | | | PTSD= 13 | Hispanic = 2 | | |
| | | | MOOD= 4 | | | |
| | | | PSYCH= 2 | | | |
| Independent Testing Cohort For Predicting Trait (Future ED visits for Pain in All Years Following Assessment) | 189 (501 visits) | Male = 170 | BP = 49 | EA= 124 | 51.79 (6.75) Others 51.58 ED visits for Pain 52.02 | ED visits for Pain (n=239) vs. Others (n=262) **0.4720** |
| | | | MDD= 34 | | | |
| | | | SZA= 45 | | | |
| | | Female = 19 | SZ= 40 | AA= 62 | | |
| | | | PTSD=15 | Hispanic=3 | | |
| | | | MOOD= 4 | | | |
| | | | PSYCH= 2 | | | |

**Table 4. Top Biomarkers for Pain**

| **Gene Symbol/Gene Name Name** | **Probeset** | **Discovery** (Change) Method/S core | Prior Human Genetic Evidence | Prior Human Nervous Tissue Evidence | Prior Human Peripheral Evidence | Prior Non-human Genetic Evidence | Prior Non-human Nervous Tissue Evidence | Prior Non-human Peripheral Evidence | **Prioritization** Total CFG Score For Pain | **Validation** Anova p-value |
|---|---|---|---|---|---|---|---|---|---|---|
| HLA-DQB1 Major Histocompatibility Complex, Class II, DQ Beta 1 | 212998_x_at | (I) | | (D) DRG Neurological Pain ¹ | (D)Blood Neurological Pain ² | | (I) Spinal Cord Neuropathic Pain ³ | | 12 | NS |
| | | DE/4 | | | | | | | | |
| | | 51% | | | | | | | | |
| HLA-DQB1 Major Histocompatibility Complex, Class II, DQ Beta 1 | 211656_x_at | (I) | | (D) DRG Neurological Pain ¹ | (D) Blood Neurological Pain ² | | (I) Spinal Cord Neuropathic Pain ³ | | 12 | NS |
| | | DE/4 | | | | | | | | |
| | | 59% | | | | | | | | |
| CAL.CA Calcitonin Related Polypeptide Alpha | 210727_at | (D) | Analgesia⁴ | | (D) Vertebral disc, Neurological Pain ⁶ | | (I) DRG Pain ⁹ | (I) blood Acute Pain 12 | 11 | NS |
| | | | | | (D) Blood Neuropathic Pain⁷ | | (I) Neurological Pain ¹⁰ | | | |
| | | DE/4 | Migraine ⁵ | | | | | | | |
| | | 54% | | | | | (I) Dorsal Horn Neurological Pain ¹¹ | | | |
| | | | | | (I) Migraine/Heada che ⁸ | | | | | |
| CCDC144B Coiled-Coil Domain Containing 144B (Pseudogene) | 1557366_at | (D) | | (I) Neurological Pain ¹ | | | (D) NAC Neuropathic Pain 13 | | 10 | NS |
| | | DE/4 | | | | | | | | |
| | | 56% | | | | | | | | |
| CNTN1 Contactin 1 | 1554784_at | (D) | | (D) DRG Neuropathy¹⁴ | (D) CSF¹⁵ | | | | 10 | NS |
| | | DE/4 | | | | | | | | |
| | | 52% | | | | | | | | |
| GNG7 G Protein Subunit Gamma 7 | 1566643_a_at | (D) | | (I) sural nerve Diabetic Neuropathy ¹⁶ | (I) vertebral disc | | | | 10 | 6.81E-02 Stepwise |
| | | DE/4 | | | | | | | | |
| | | 59% | | | | | | | | |
| | | | | | Neurological Pain ⁶ | | | | | |
| **HLA-DQB1** Major Histocompatibility Complex, Class II, DQ Beta 1 | 210747_at | (D) | | (D) DRG Neurological Pain ¹ | (D) Whole blood Neurological Pain ² | | (I) Spinal Cord Neuropathic Pain ³ | | 10 | NS |
| | | DE/2 | | | | | | | | |
| | | 44% | | | | | | | | |
| **HLA-DQB1** Major Histocompatibility Complex, Class II, DQ Beta 1 | 211654_x_at | (I) | | (D) DRG Neurological Pain ¹ | (D) Whole blood Neurological, Pain ² | | (I) Spinal Cord Neuropathic Pain ³ | | 10 | NS |
| | | DE/2 | | | | | | | | |
| | | 40% | | | | | | | | |
| **ASTN2** Astrotactin 2 | 1554816_at | (I) | Chronic Migraine ¹⁷, ¹⁸, ¹⁹, ²⁰ | | | | | | 8 | 1.71E-01 Stepwise |
| | | DE/6 | | | | | | | | |
| | | 83% | | | | | | | | |
| **CASP6** Caspase 6 | 209790_s_at | (I) | | | (I) vertebral disc Neurological ⁶ | | DRG Neuropathic pain ²¹ | | 8 | NS |
| | | DE/4 | | | | | | | | |
| | | 51% | | | | | | | | |
| **CCDC85C** Coiled-Coil Domain Containing 85C | 219018_s_at | (D) | | | | | (I) PAG Neuropathic Pain ¹³ | | 8 | NS |
| | | DE/6 | | | | | | | | |
| | | 94% | | | | | | | | |
| **CCND1** Cyclin D1 | 208712_at | (D) | | | (D) Serum Chronic Pain ²² | | (I) (DRG) Neurological Pain ¹⁰ | | 8 | NS |
| | | DE/4 | | | | | | | | |
| | | 57% | | | | | | | | |
| **CDK6** Cyclin Dependent Kinase 6 | 224851_at | (I) | | | (D) Serum Chronic Pain ²² | | (I) Neuropathic Pain ²³ | | 8 | NS |
| | | DE/4 | | | | | | | | |
| | | 56% | | | | | | | | |
| | | (I) | | | | | | | | |
| | | AP/2 | | | | | | | | |
| | | 42% | | | | | | | | |
| **CDK6** Cyclin Dependent Kinase 6 | 224847_at | (I) | | | (D) Serum Chronic Pain ²² | | (I) Neuropathic Pain ²³ | | 8 | NS |
| | | DE/4 | | | | | | | | |
| | | 63% | | | | | | | | |
| **COL27A1** Collagen Type XXVII Alpha 1 Chain | 225293_at | (D) | | | (D) Lymphoblast Migraine ²⁴ | | (I) PAG Neuropathic Pain ¹³ | | 8 | 7.47E-01 Stepwise |
| | | D | | | | | | | | |
| | | E/4 79% | | | | | | | | |
| **COL2A1** Collagen Type II Alpha 1 Chain | 217404_s_at | (D) | | | (I) vertebral disc Neurological Pain ⁶ | | (I) PAG Chronic Neuropathic Pain ¹³ | | 8 | NS |
| | | DE/4 | | | | | | | | |
| | | 54% | | | | | | | | |
| **COMT** Catechol-O-Methyltransferase | 216204_at | | Neurological Pain ²⁵, ²⁶ | | (D) Blood Chronic Pain, MSK ⁴² | | | | 8 | NS |
| | | | Chronic Pain MSK ^{27 28, 29 30, 31,32,33,34,35,36,37} | | | | | | | |
| | | (D) | Pain, Acute, Thermal ³⁸ | | | | | | | |
| | | DE/4 | Treatments ³⁹ | | | | | | | |
| | | 54% | | | | | | | | |
| | | | Pain MSK ²⁹,²⁸,²⁷ | | | | | | | |
| | | | Pain ⁴⁰ | | | | | | | |
| | | | Morphine ⁴¹ | | | | | | | |
| **COMT** Catechol-O-Methyltransferase | 213981_at | (D) | Neurological Pain ²⁵, ²⁶ | | (D) blood Chronic Pain, MSK ⁴² | | | | 8 | NS |
| | | DE/4 | Chronic Pain MSK ^{27 28, 29 30, 31,32,33,34,35,36,37} | | | | | | | |
| | | 54% | | | | | | | | |
| | | | Pain, Acute, Thermal ³⁸ | | | | | | | |
| | | | Treatments ³⁹ | | | | | | | |
| | | | Pain MSK ²⁹,²⁸,²⁷ | | | | | | | |
| | | | Pain ⁴⁰ | | | | | | | |
| | | | Morphine ⁴¹ | | | | | | | |
| **DCAF12** DDB1 And CUL4 Associated Factor 12 | 224789_at | (D) | | | (I) Whole blood Neurological, Pain ² | | | | 8 | NS |
| | | DE/6 | | | | | | | | |
| | | 86% | | | | | | | | |
| **EDN1** Endothelin 1 | 1564630_at | (I) | Fibromyalgia ⁴³ | | (I) Blister fluid Chronic Pain ⁴⁴ | | | | 8 | 8.69E-02 Stepwise |
| | | AP/4 | | | | | | | | |
| | | 56% | | | | | | | | |
| **FAM134B** Family With Sequence Similarity 134 Member B | 218510_x_at | (I) | Chronic, Neuropathic Pain ⁴⁵ | | (I) vertebral disc Neurological Pain ⁶ | | | | 8 | NS |
| | | DE/4 | | | | | | | | |
| | | 51%; (I) | | | | | | | | |
| | | AP/2 | | | | | | | | |
| | | 34% | | | | | | | | |
| **GBP1** Guanylate Binding Protein 1 | 231578_at | (I) | Fibromyalgia ⁴⁶ | (D) Neurological Pain ¹ | | | | | 8 | 3.26E-01 Stepwise |
| | | DE/2 | | | | | | | | |
| | | 37% | | | | | | | | |
| **HLA-DRB1** Major Histocompatibility Complex, Class II, DR Beta 1 | 208306_x_at | (I) | Migraine⁴⁷ | | (I) Whole blood Neurological Pain ² | | | | 8 | NS |
| | | AP/4 | | | | | | | | |
| | | 52% | | | | | | | | |
| **HTR2A** 5-Hydroxytryptamin e Receptor 2A | 211616_s_at | | Neurological, Pain ⁴⁸ | | (D) whole blood, Neuropathic ⁷ | | | | 8 | NS |
| | | | Chronic, MSK ³¹,⁴⁹,⁵⁰ | | | | | | | |
| | | (D) | Fibromyalgia ⁵¹,⁵², ⁵³ | | | | | | | |
| | | DE/4 | | | | | | | | |
| | | 52% | Pain, Acute, disease/lesion ⁵⁴ | | | | | | | |
| | | | Pain ⁴⁰,⁵⁵ | | | | | | | |
| **LOXL2** Lysyl Oxidase Like 2 | 228808_s_at | (D) | | | (I) vertebral disc Neurological Pain ⁶ | | (I) PFC Chronic Neuropathic Pain ¹³ | | 8 | NS |
| | | DE/4 | | | | | | | | |
| | | 59% | | | | | | | | |
| **LY9** Lymphocyte Antigen 9 | 231124_x_at | (I) | | | | | (D) NAC Chronic Neuropathic Pain ¹³ | | 8 | NS |
| | | DE/6 | | | | | | | | |
| | | 90% | | | | | | | | |
| **NF1** Neurofibromin 1 | 212676_at | (I) | Migraine ⁵⁶ | | (I) vertebral disc Neurological Pain ⁶ | | | | 8 | NS |
| | | DE/4 | | | | | | | | |
| | | 59% | | | | | | | | |
| **PNOC** Prepronociceptin | 205901_at | (I) | | | (D) vertebral disc Neurological Pain ⁶ | | (I) PAG Chronic Neuropathic Pain ¹³ | | 8 | NS |
| | | DE/4 | | | | | | | | |
| | | 62% | | | (I) whole blood Neuropathic Pain ⁷ | | | | | |
| **SHMT1** Serine Hydroxymethyltra nsferase 1 | 217304_at | (D) | Musculoskeletal Pain ⁵⁷ | (D) Neurological Pain ¹ | | | | | 8 | NS |
| | | DE/2 | | | | | | | | |
| | | 43% | | | | | | | | |
| T**CF15** Transcription Factor 15 (Basic Helix-Loop-Helix) | 207306_at | (D) | | | | | (I) PFC Chronic Neuropathic Pain ¹³ | | 8 | NS |
| | | DE/6 | | | | | | | | |
| | | 94% | | | | | | | | |
| **TOP3A** Topoisomerase (DNA) III Alpha | 214300_s_at | (D) | | (D) Neurological Pain ¹ | | | | | 8 | NS |
| | | DE/4 | | | | | | | | |
| | | 51% | | | | | | | | |
| **TSPO** Translocator Protein | 202096_s_at | (I) | Neuraxial Pain⁵⁸ | | (I) vertebral disc Neurological Pain ⁶ | | (I) PAG Neuropathic Pain ¹³ | | 8 | NS |
| | | DE/2 | | | | | | | | |
| | | 38% | | | | | (I) DRG) Neurological Pain ¹⁰ | | | |
| **VEGFA** Vascular Endothelial Growth Factor A | 212171_x_at | (I) | Neuraxial Pain⁵⁹ | | (I) Blood Steroid ⁶⁰ | | | | 8 | NS |
| | | AP/4 | | | (I) Chronic Pain ⁶¹ | | | | | |
| | | 65% | | | | | | | | |
| | | | | | (I) serum Acute Pain MSK ⁶² | | | | | |
| **WNK1** WNK Lysine Deficient Protein Kinase 1 | 1555068_at | (D) | Chronic Neuropathic Pain ⁶³ | | | | | | 8 | NS |
| | | DE/6 | | | | | | | | |
| | | 92% | Pain ⁴⁰ | | | | | | | |
| **ZNF429** Zinc Finger Protein 429 | 1561270_at | (D) | Pain MSK ⁶⁴ | (I) Neurological Pain ¹ | | | | | 8 | NS |
| | | DE/2 | | | | | | | | |
| | | 37% | Analgesia ⁶⁵ | | | | | | | |
| **ZYX** Zyxin | 238016_s_at | (D) | | | (I) Whole blood Neurological Pain ² | | (I) PAG Chronic Neuropathic Pain ¹³ | | 8 | NS |
| | | DE/4 | | | | | | | | |
| | | 57% | | | | | | | | |
| **(AF087971) PBRM1** Polybromo 1 | 1561067_at | (I) | | | | | | | 6 | NS |
| | | AP/6 | | | | | | | | |
| | | 90% | | | | | | | | |
| **(AF090920) PPFIBP2** PPFIA Binding Protein 2 | 234739_at | (I) | | | | | | | 6 | NS |
| | | AP/6 | | | | | | | | |
| | | 94% | | | | | | | | |
| **(H05785) LRRC75A** Leucine Rich Repe at Containing 75A | 236913_at | (D) | | | | | | | 6 | NS |
| | | AP/6 | | | | | | | | |
| | | 97% | | | | | | | | |
| **(Hs.596713) PPP1R14B** Protein Phosphatase 1 Regulatory Inhibitor Subunit 14B | 226138_s_at | (D) | | | | | | | 6 | 6.28E-02 Stepwise |
| | | DE/6 | | | | | | | | |
| | | 90% | | | | | | | | |
| **(Hs.609761) SFPQ** Splicing Factor Proline And Glutamine Rich | 244331_at | (D) | | | | | | | 6 | NS |
| | | DE/6 | | | | | | | | |
| | | 98% | | | | | | | | |
| **(Hs.659426) PHC3** Polyhomeotic Homolog 3 | 240599_x_at | (D) | | | | | | | 6 | NS |
| | | DE/6 | | | | | | | | |
| | | 92% | | | | | | | | |
| **(Hs.666804) MFAP3 Microfibril Associated Protein 3** | 240949_x_at | (D) | | | | | | | 6 | ***6.03E-04 Nominal*** |
| | | DE/6 | | | | | | | | |
| | | 81% | | | | | | | | |
| **(Hs.677263) SMURF2** (SMAD Specific E3 Ubiquitin Protein Ligase 2) | 216444_at | (D) | | | | | | | 6 | NS |
| | | AP/6 | | | | | | | | |
| | | 100% | | | | | | | | |
| | | (D) | | | | | | | | |
| | | D E/4 | | | | | | | | |
| | | 71% | | | | | | | | |
| **(Hs.696420) MTERF1** | 243125_x_at | (D) | | | | | | | 6 | NS |
| | | DE/6 | | | | | | | | |
| | | 100% | | | | | | | | |
| Mitochondrial Transcription Termination Factor 1 | | | | | | | | | | |
| **CLSPN** Claspin | 242150_at | (I) | | | | | | | 6 | NS |
| | | AP/6 | | | | | | | | |
| | | 95% | | | | | | | | |
| **DENND1B** DENN Domain Containing 1B | 1557309_at | (I) | | | | | | | 6 | NS |
| | | DE/6 | | | | | | | | |
| | | 90%; (I) | | | | | | | | |
| | | AP/2 | | | | | | | | |
| | | 40% | | | | | | | | |
| **DNAJC18** DnaJ Heat Shock Protein Family (Hsp40) Member C18 | 227166_at | (I) | | | | | | | 6 | NS |
| | | DE/6 | | | | | | | | |
| | | 94% | | | | | | | | |
| **ELAC2 ElaC Ribonuclease Z 2** | 201766_at | (D) | Fibromyalgia⁶⁶ | | | | | | 6 | **4.11E-02 *Nominal*** |
| | | DE/4 | | | | | | | | |
| | | 52% | | | | | | | | |
| **GSPT1** G1 To S Phase Transition 1 | 215438_x_at | (D) | | | | | | | 6 | NS |
| | | DE/6 | | | | | | | | |
| | | 94% | | | | | | | | |
| **HRAS** HRas Proto-Oncogene, GTPase | 212983_at | (I) | | | | | | | 6 | NS |
| | | DE/6 | | | | | | | | |
| | | 97% | | | | | | | | |
| **Hs.554262** | 210703_at | (I) | | | | | | | 6 | NS |
| | | AP/6 | | | | | | | | |
| | | 100% | | | | | | | | |
| **MBNL3** Muscleblind Like Splicing Regulator 3 | 219814_at | (D) | | | | | | | 6 | NS |
| | | DE/6 | | | | | | | | |
| | | 92% | | | | | | | | |
| **MCRS1** Microspherule Protein 1 | 202556_s_at | (I) | | | | | | | 6 | NS |
| | | DE/6 | | | | | | | | |
| | | 90% | | | | | | | | |
| **OSBP2** Oxysterol Binding Protein 2 | 1569617_at | (D) | | | | | | | 6 | NS |
| | | DE/6 | | | | | | | | |
| | | 94% | | | | | | | | |
| **PIK3CD Phosphatidylinosit ol-4,5-Bisphosphate 3-Kinase Catalytic Subunit Delta** | 211230_s_at | (D) | | | | | | | 6 | ***1.59E-02 Nominal*** |
| | | DE/6 | | | | | | | | |
| | | 83% | | | | | | | | |
| **PTN** Pleiotrophin | 211737_x_at | (D) | | | | | | | 6 | NS |
| | | DE/6 | | | | | | | | |
| | | 92% | | | | | | | | |
| **RAB33A** RAB33A, Member RAS Oncogene Family | 206039_at | (I) | | | | | | | 6 | NS |
| | | DE/6 | | | | | | | | |
| | | 90% | | | | | | | | |
| **RALGAPA2** Ral GTPase Activating Protein Catalytic Alpha Subunit 2 | 231826_at | (D) | | | | | | | 6 | NS |
| | | DE/6 | | | | | | | | |
| | | 97% | | | | | | | | |
| **SEPT7P2** Septin 7 Pseudogene 2 | 1569973_at | **(I)** | | | | | | | 6 | NS |
| | | **DE/6** | | | | | | | | |
| | | **100%** | | | | | | | | |
| | | (I) | | | | | | | | |
| | | AP/2 | | | | | | | | |
| | | 39% | | | | | | | | |
| **SVEP1 Sushi, Von Willebrand Factor Type A, EGF And Pentraxin Domain Containing 1** | 236927_at | (I) | Migraine ⁵⁶ | | | | (D) NAC Neuropathic Pain ¹³ | | 6 | ***2.17E-02 Nominal*** |
| | | DE/2 | | | | | | | | |
| | | 49% | | | | | | | | |
| **TNFRSF11B TNF Receptor Superfamily Member 11b** | 204932_at | (D) | Cancer Pain ⁶⁷ | | (I) vertebral disc Neurological Pain ⁶ | | | | 6 | ***2.67E-02 Nominal*** |
| | | DE/2 | | | | | | | | |
| | | 37% | | | (I) Serum Chronic Pain 68 | | | | | |
| **YBX3** Y-Box Binding Protein 3 | 201160_s_at | (D) | | | | | | | 6 | NS |
| | | DE/6 | | | | | | | | |
| | | 94% | | | | | | | | |
| **ZNF441** Zinc Finger Protein 441 | 1553193_at | (I) | | | | | | | 6 | NS |
| | | AP/6 | | | | | | | | |
| | | 95% | | | | | | | | |
| | | (I) | | | | | | | | |
| | | DE/2 | | | | | | | | |
| | | 35% | | | | | | | | |
| **ZNF91** Zinc Finger Protein 91 | 244259_s_at | (I) | | | | | | | 6 | 6.37E-01/2 Stepwise |
| | | AP/6 | | | | | | | | |
| | | 95% | | | | | | | | |

| | | | | | | | | | | |
|---|---|---|---|---|---|---|---|---|---|---|
| (n=60 genes, 65 probesets) - evidence for involvement in pain. (I) - increased in expression in Pain, (D)- decreased in expression. DE-differential expression, AP-Absent/Present. DRG- dorsal root ganglia. | | | | | | | | | | |

**Table 5. Top biomarkers for pain - Evidence for involvement in other psychiatric and related disorders.**

| **Gene Symbol/G ene Name Name** | **Probe set** | **Disc over y (Cha nge) Meth od/S core** | **Prioritization Total CFG Score For Pain** | **Validat ion Anova p-value** | **Prior human genetic evidence for other disorders 2 pts.** | **Prior human Brain expression evidence for other disorders 4 pts** | **Prior human peripheral evidence for other disorders 2pts** | **Prior Non-human genetic evidence for other disorders 1pt.** | **Prior Non-human Brain expression evidence for other disorders 2pts.** | **Prior Non-human periph eral eviden ce for other disorde rs 1pt.** | **Exter nal CFG for Other Dx** |
|---|---|---|---|---|---|---|---|---|---|---|---|
| **HTR2A** 5-Hydroxytr yptamine Receptor 2A | 2116 16_s_ at | (D) | 8 | NS | Alcoholism ⁶⁹ BP ^{70 71 72 70, 73, 74} | (D) HIP BP ⁹¹ | (D) Lymphocyte SZ ¹⁰³ | Anxiety ¹⁰⁶ | (D) PFC SZ ¹⁰⁷ | | 13 |
| | | | | | | (D) HIP SZ, Depression⁹² | | | | | |
| | | | | | Depression ⁷⁵⁻⁷⁷ 78 | | | | | | |
| | | D E/4 | | | | (D) DLPFC BP ⁹² | | | | | |
| | | 52% | | | Mood ⁷⁹ | | | | (D) Frontal cortex Depression, SZ ¹⁰⁸ | | |
| | | | | | OCD ⁸⁰ | (D) Temporal Cortex SZ ⁹³ | (D) PBMC SZ ¹⁰⁴ | | | | |
| | | | | | Addictions ^{81, 82 83 84 85} | | | | | | |
| | | | | | Suicide ^{79, 86 87-90} | (D) HIP BP, SZ⁹⁴Suicide ⁹⁵ | (D) Platelets Suicide ¹⁰⁵ | | (D) PFC Hallucinogens ¹⁰⁹ | | |
| | | | | | | (D) PFC Aging ⁹⁶ | | | | | |
| | | | | | | (D) frontal cortex Suicide ⁹⁷ | | | (D) AMY PTSD ¹¹⁰ | | |
| | | | | | | | | | (I) AMY Depression¹¹¹ | | |
| | | | | | | (D) BA46 Suicide ⁹⁸ | | | | | |
| | | | | | | (D) Brain BP ⁹⁹ | | | | | |
| | | | | | | (I) AMY,Frontopola r cortex Suicide ¹⁰⁰ | | | | | |
| | | | | | | (D) PFC SZ¹⁰¹ | | | | | |
| | | | | | | (D) DLFPC Suicide ¹⁰² | | | | | |
| **CDK6** Cyclin Dependen t Kinase 6 | 2248 47_at | (I) | 8 | NS | Circadian abnormalities ¹¹² | (I) PFC SZ ¹¹⁶ | (I) lymphoblast oid ASD ¹¹⁸ | | (I) AMY MDD ¹²¹ | | 10 |
| | | | | | | | (I)Blood Female Suicide ¹¹⁹ | | | | |
| | | DE/4 | | | | (I) Brain SZ ¹¹⁷ | | | | | |
| | | 63% | | | Longevity ¹¹³,¹¹⁴ | | | | | | |
| | | | | | Alcohol ¹¹⁵ | | (I) Blood M-BP Suicide ¹²⁰ | | | | |
| **CDK6** Cyclin Dependen t Kinase 6 | 2248 51_at | (I) | 8 | NS | Circadian abnormalities ¹¹² | (I) PFC SZ ¹¹⁶ | (I) lymphoblast oid ASD ¹¹⁸ | | (I) AMY MDD ¹²¹ | | 10 |
| | | DE/4 | | | | | | | | | |
| | | 56% | | | | | (I)Blood Female Suicide ¹¹⁹ | | | | |
| | | (I) | | | Longevity ¹¹³, ¹¹⁴ | (I) Brain SZ ¹¹⁷ | | | | | |
| | | AP/2 | | | Alcohol ¹¹⁵ | | (I) Blood M-BP Suicide ¹²⁰ | | | | |
| | | 42% | | | | | | | | | |
| **HLA-DQB1** Major Histocomp atibility Complex, Class II, DQ Beta 1 | 2129 98_x_ at | | 8 | NS | Longevity ^{122 ,123} | (I) Superior temporal cortex (BA 22) SZ ¹²⁶ | (I) Blood SZ ¹²⁷ | | (I) CP, NAC | | 10 |
| | | (I) | | | | | (I) Blood Suicide^{128 129} | | | | |
| | | DE/4 | | | | | | | | | |
| | | (I) | | NS | | | (I) PBMC ¹³⁰ | | (D) AMY Alcoholism ¹³³ | | |
| | 2116 56_x_ at | DE/4 | | | SZ ^{124, 125} | | Stress | | | | |
| | | 59% | | | | | PTSD¹³¹ | | | | |
| | | | | | | | (I) Leukocytes Depression^{13 2} | | | | |
| **WNK1** WNK Lysine Deficient Protein Kinase 1 | 1555 068_a t | (D) | 8 | NS | Depression ¹³⁴ | (D) NAC Alcohol ¹³⁵ | (D) Blood Suicide ¹²⁹, ¹²⁰ | | (D) PFC (male) BP, Stress ¹³⁶ | | 10 |
| | | DE/6 | | | | | | | | | |
| | | 92% | | | | | | | | | |
| **(AF087971) PBRM1** Polybromo 1 | 1561 067_a t | | 6 | NS | CNV,MDD ¹³⁷ BP ^{138-140 141-143} Mood, | (I) DLPFC BP ¹³⁹ | (I) Blood Hallucination s¹⁴⁷ | | (I) AMY MDD¹²¹ | | 10 |
| | | (I) | | | | | (I) Blood Mood ¹⁴⁸ | | (I) AMY(male) BP, Stress ¹³⁶ | | |
| | | AP/6 | | | Psychosis ^{144 139} | | (I) Blood Male Suicide ¹²⁹ | | | | |
| | | 90% | | | Depression MDD ^{139 140} SZ ^{141, 145} | | | | | | |
| | | | | | | | | | (I) Brain Stimulants ¹⁴⁹ | | |
| | | | | | Longevity¹⁴⁶ | | (I) Blood Female Suicide ¹¹⁹ | | | | |
| **(Hs.66680 4) MFAP3 Microfibril Associated Protein 3** | 2409 49_x_ at | (D) | 6 | ***6.03E-04*/*4 Nomin al*** | SZ ¹²⁴ | (D)Superior frontal cortex Alcohol ¹⁵⁰ | (D)Blood Suicide ¹²⁹,¹²⁰ | | (D) AMY Stress ¹²¹ | | 10 |
| | | DE/6 | | | | | | | | | |
| | | 81% | | | | | | | | | |
| **CCND1** Cyclin D1 | 2087 12_at | (D) | 8 | NS | | (D) Frontal motor cortex Alcohol ¹⁵¹ | (D) Peripheral blood Stress ¹⁵⁴ | Addiction Alcohol ¹⁵⁵ | (D) Amygdala) Hallucinogens ¹⁵⁶ | | 9 |
| | | DE/4 | | | | | | | | | |
| | | 57% | | | | | | | | | |
| | | | | | | (D) hippocampus Alcohol ¹⁵² | | | (D) Amygdala Addiction Alcohol ¹³³ | | |
| | | | | | | (D) ACC MDD ¹⁵³ | | | | | |
| **CNTN1** Contactin 1 | 15547 84_at | | 10 | NS | BP,SZ ¹⁵⁷; ^{158 134}MDD | (D) Brain BP ⁹⁹ | (D) lymphocyte SZ ¹⁶⁴ | | | | 8 |
| | | | | | | (D) HIP BP ¹⁶⁰ | | | | | |
| | | (D) | | | | (D) Forebrain neural progenitor cells SZ ¹⁶¹ | | | | | |
| | | DE/4 | | | Suicide¹⁵⁹ | | (D) Blood Female Suicide ¹¹⁹ | | | | |
| | | 52% | | | | (D) supragenual | | | | | |
| | | | | | | (BA24) anterior cingulated cortex SZ ¹⁶² | | | | | |
| | | | | | | (D) anterior PFC SZA ¹⁶³ | | | | | |
| **GBP 1** Guanylate Binding Protein 1 | 2315 78_at | (I) | 8 | 3.26E-01/2 Stepwi se | | (I) Hippocampus, amygdala, gyrus cinguli ,pons MDD ¹⁶⁵ (I) amygdala ¹⁶⁶ | (I) leukocytes PTSD ¹⁶⁸ | | (I) hippocampal and prefrontal cortex MDD ¹⁶⁵ | | 8 |
| | | DE/2 | | | | | | | | | |
| | | 37% | | | | SZ (I) left side superior frontal gyrus SZ ¹⁶⁷ | | | | | |
| | | | | | | (I) Brain Suicide ¹⁶⁵ | | | | | |
| **HLA-DQB1** Major Histocomp atibility Complex, Class II, DQ Beta 1 | 2116 54_x_ at | (I) | 8 | NS | | (I) superior temporal cortex SZ ¹²⁶ | (I) monocytes Stress ¹³⁰ | | (I) Caudate putamen Addiction Alcohol¹³³ | | 8 |
| | | DE/2 | | | | | | | | | |
| | | 40% | | | | | (I) PBMC PTSD ¹³¹ | | | | |
| **PNOC** Prepronoci ceptin | 2059 01_at | (I) | 7 | NS | Addictions ¹⁶⁹ | (I) DLPFC BP,SZ ¹⁷⁰ | (I) Fibroblasts SZ ¹⁷² | | (I) NAC Stress ¹⁷³ | | 8 |
| | | DE/4 | | | | | | | | | |
| | | 62% | | | | | | | (I) Amygdala MDD ¹¹¹ | | |
| | | | | | | (I) AMY, cingulate cortex MDD ¹⁷¹ | | | | | |
| | | | | | | (I) Forebrain neural cells SZ ¹⁶¹ | | | | | |
| **GSPT1** G1 To S Phase Transition 1 | 2154 38_x_ at | (D) | 6 | NS | | (D) Brain BP ⁹⁹ | (D) Blood Suicide ¹²⁹ | | (D) AMY Depression¹²¹ | | 8 |
| | | DE/6 | | | | | (D) Leukocytes Depression^{13 2} | | | | |
| | | 94% | | | | | | | | | |
| **(Hs.60976 1) SFPQ** Splicing Factor Proline And Glutamine Rich | 2443 31_at | (D) | 6 | NS | | NAC altered MDD ¹⁷⁴ | (D) Blood Female Suicide ¹¹⁹ | | (D) VT Hallucinogens ¹⁵⁶ | | 8 |
| | | | | | | | | | (D) PFC (male) Stress, BP ¹³⁶ | | |
| | | DE/6 | | | | | | | | | |
| | | 98% | | | | (D) superior frontal cortex Alcohol ¹⁵⁰ | | | | | |
| | | | | | | (D) PFC MDD ¹⁷⁵ | | | (D) Brain Alcohol Addiction¹⁷⁶ | | |
| **ZNF91** Zinc Finger Protein 91 | 2442 59_s_ at | (I) | 6 | 6.37E-01/2 Stepwis e | Circadian abnormalities ¹¹² | (I) Temporal cortex | (I) Blood PTSD ¹⁷⁹ | | | | 8 |
| | | AP/6 | | | | Alcoholism ¹⁷⁷ | | | | | |
| | | 95% | | | | (I) DLPFC PTSD ¹⁷⁸ | | | | | |
| **COMT** Catechol-O-Methyltra nsferase | 2162 04_at 2139 81_at | (D) | 8 | | OCD ^{180 181 182 183-185 186} | | (D) Blood Alcoholism ²²⁷ | SZ^{230, 231} | (D) PFC Anxiety, OCD, SZ²³² | | 7 |
| | | | | NS | BP ^{187 182 188 189, 190 191, 192 194} | | (D) Blood SZ ²²⁸ | | (D) Brain Anxiety ²³³ | | |
| | | D E/4 | | | | | | | | | |
| | | 54% | | NS | | | | Alcoholism ¹⁵⁵ | | | |
| | | (D) | | | Anxiety^{193 195 196 197 198 199 200 201} | | | | | | |
| | | D E/4 | | | | | (D) Leukocytes SZ ²²⁹ | | (D) Male HIP, AMY Anxiety ²³⁴ | | |
| | | | | | SZ ^{202 203 204 205 206 207 192,} | | | | | | |
| | | | | | ^{208 209} | | | | | | |
| | | | | | Aggression ^{210 211 212} | | (D) PBMC Stress ¹³⁰ | | | | |
| | | | | | Suicide ^{213 214 215 216 217} | | (D) Blood Suicide¹¹⁹ | | | | |
| | | | | | Thermal ²¹⁸ | | | | | | |
| | | | | | Stimulants²¹⁹ | | | | | | |
| | | | | | Intellect²²⁰ | | | | | | |
| | | | | | Mood ²⁰⁹ | | | | | | |
| | | | | | ADHD ¹⁸⁶ | | | | | | |
| | | | | | Depression^{78 221-223} | | | | | | |
| | | | | | PTSD ^{224 225} | | | | | | |
| | | | | | Alcohol²²⁶ | | | | | | |
| **VEGFA** Vascular Endothelia l Growth Factor A | 2121 71_x_ at | (I) | 8 | NS | | (I) CA3/2 Stratum oriens SZ ²³⁵ | (I) monocytes Stress ¹³⁰ | MDD ²⁴⁰ | | | 7 |
| | | AP/4 | | | | (I) Prefrontal cortex SZ ²³⁶ | (I) plasma MDD ²³⁸ | | | | |
| | | 65% | | | | | | | | | |
| | | | | | | (I) hippocampus SZ ²³⁷ | (I) plasma BP ²³⁹ | | | | |
| **(H05785) LRRC75A** Leucine Ri ch Repeat Containin g 75A | 2369 13_at | (D) | 6 | NS | | (D) Brain BP ⁹⁹ | (D) Blood Male BP | Alcohol Addiction¹⁵⁵ | | | 7 |
| | | AP/6 | | | | (D) DLPFC SZ ²⁴¹ | | | | | |
| | | 97% | | | | | Suicide ¹²⁰ | | | | |
| **CALCA** Calcitonin Related Polypeptid e Alpha | 2107 27_at | (D) | 7 | NS | | (D) Frontal, motor cortex | | | | | 6 |
| | | DE/4 | | | | | | | (D) Medullae | | |
| | | 54% | | | | Alcohol¹⁵¹ | | | Oblongata Anxiety ²⁴² | | |
| **LOXL2** Lysyl Oxidase Like 2 | 2288 08_s_ at | (D) | 7 | NS | | (D) anterior PFC BP ¹⁶³ | (D) Male-BP Suicide¹²⁰ | | | | 6 |
| | | DE/4 59% | | | | | | | | | |
| | | | | | | | | | | | |
| **HRAS** HRas Proto-Oncogene, GTPase | 2129 83_at | (I) | 6 | NS | BP, SZ ¹⁵⁷ | | | | (I) NAC Alcohol ²⁴⁵ | | 6 |
| | | DE/6 | | | | | mRNA | | | | |
| | | 97% | | | Longevity ²⁴³ | | Suicide ²⁴⁴ | | | | |
| **(Hs.696420 ) MTERF1** Mitochond rial Transcript! on Terminati on Factor 1 | 2431 25_x_ at | (D) | 6 | NS | | (D) DPFC BA ⁴⁶ PTSD ²⁴⁶ | (D) Blood Universal Suicide ¹²⁰ | | | | 6 |
| | | DE/6 | | | | | | | | | |
| **PIK3CD Phosphati dylinositol -4,5-Bisphosph ate 3-Kinase Catalytic Subunit Delta** | 2112 30_s_ at | (D) | 6 | ***1.59E-0214 Nomin al*** | Longevity ²⁴⁷ | | (D) PBMC Stress ¹³⁰ | | (D) NAC Alcohol ¹³³ | | 6 |
| | | DE/6 | | | | | (D) Blood Suicide ¹²⁰ mRNA Suicide ²⁴⁴ | | | | |
| | | 83% | | | SZ ²⁴⁸ | | | | | | |
| **PTN** Pleiotrophi n | 2117 37_x_ at | (D) | 6 | NS | SZ ^{145 249} | | mRNA Suicide ²⁴⁴ | | | | 6 |
| | | DE/6 | | | | | | | (D) HIP | | |
| | | 92% | | | | | | | Stress²⁵⁰ | | |
| **YBX3** Y-Box Binding Protein 3 | 2011 60_s_ at | (D) | 6 | NS | | (D) DLPFC BP,SZ ¹⁷⁰ | (D) Blood Male Suicide ¹²⁹ | | | | 6 |
| | | DE/6 | | | | | | | | | |
| | | 94% | | | | | | | | | |
| **NF1** Neurofibro min 1 | 2126 76_at | (I) | 8 | NS | | Differentially expressed ACC (BA 24) BP ²⁵¹ | | Addiction Alcohol ¹⁵⁵ | (I) VS PTSD ¹¹⁰ | | 5 |
| | | DE/4 | | | | | | | | | |
| | | 59% | | | | | | | | | |
| **SVEP1 Sushi, Von Willebrand Factor Type A, EGF And Pentraxin Domain** | 2369 27_at | (1) | 6 | ***2.17E-0214 Nomin al*** | | (I) Hippocampus SZ ²⁵² | | Alcohol ¹⁵⁵ | | | 5 |
| | | DE/2 | | | | | | | | | |
| | | 49% | | | | | | | | | |
| **Containin g 1** | | | | | | | | | | | |
| **(Hs.67726 3)Smurf2** SMAD Specific E3 Ubiquitin Protein Ligase 2 | 2164 44_at | (D) | 6 | NS | | | (D) Blood Suicide ¹²⁹,¹²⁰ | | (D) VM PFC Stress ²⁵³ | Interve rtebral disc Aging ²⁵⁴ | 5 |
| | | AP/6 | | | | | | | | | |
| | | 100 % | | | | | | | | | |
| | | (D) | | | | | | | | | |
| | | DE/4 | | | | | | | | | |
| | | 71% | | | | | | | | | |
| **ASTN2** Astrotacti n 2 | 1554 816_a t | | 8 | 1.71E-01 Stepwi se | Stimulants²⁵⁵ | | (I) Female Blood Suicide¹¹⁹ | | | | 4 |
| | | | | | SZ^{256 257 206} | | | | | | |
| | | (I) | | | Autism²⁵⁸ | | | | | | |
| | | DE/6 | | | Autism | | | | | | |
| | | 83% | | | CNV²⁵⁹ | | | | | | |
| | | | | | BP²⁵⁷ | | | | | | |
| **CASP6** Caspase 6 | 2097 90_s_ at | (I) | 8 | NS | | (I) Dorsolateral prefrontal cortex BP ²⁶⁰ | | | | | 4 |
| | | DE/4 | | | | | | | | | |
| | | 51% | | | | | | | | | |
| **FAM134B** Family With Sequence Similarity 134 Member B | 2185 10_x_ at | (I) | 8 | NS | Antisocial Personality ²⁶¹ | | (I) Male BP SI, Universal SI¹²⁰ | | (I) VT Hallucinogens ¹⁵⁶ | | 4 |
| | | DE/4 | | | | | | | | | |
| | | 51% ; (I) AP/2 | | | | | | | | | |
| | | 34% | | | | | | | | | |
| **HLA-DQB1** Major Histocomp atibility Complex, Class II, DQ Beta 1 | 2107 47_at | (D) | 8 | NS | | | (D) leukocytes Stress, ²⁶² | | (D) Amygdala Addictions, Alcohol ¹³³ | | 4 |
| | | DE/2 | | | | | | | | | |
| | | 44% | | | | | | | | | |
| **ZYX** Zyxin | 2380 16_s_ at | (D) | 7 | NS | | | (D) Blood MDD ²⁶³ | | (D) AMY MDD ²⁶⁴ | | 4 |
| | | DE/4 | | | | | | | | | |
| | | 57% | | | | | | | | | |
| **DNAJC18** | 2271 66_at | (I) | 6 | NS | | ACC (BA 24) BP ²⁶⁵ | | | | | 4 |
| | | DE/6 | | | | | | | | | |
| DnaJ Heat Shock Protein Family (Hsp40) Member C18 | | | | | | | | | | | |
| **MCRS1** Microsphe rule Protein 1 | 2025 56_s_ at | (I) | 6 | NS | | (I) Pituitary Depression²⁶⁶ | | | | | 4 |
| | | DE/6 | | | | | | | | | |
| **OSBP2** Oxysterol Binding Protein 2 | 1569 617_a t | (D) | 6 | NS | SZ ²⁶⁷ | | (D) Blood Suicide ^{128, 129} | | | | 4 |
| | | DE/6 | | | | | (D) SH-SY5Y cells Cocaine ²⁶⁸ | | | | |
| **RAB33A** RAB33A, Member RAS Oncogene Family | 2060 39_at | (I) | 6 | NS | | (I) Frontal Cortex Alcohol ²⁶⁹ | | | | | 4 |
| | | DE/6 | | | | (I) Stress²⁷⁰ (I)PFC,ACC,MD D¹⁵³ | | | | | |
| **TSPO** Translocat or Protein | 2020 96_s_ at | (I) | 6 | NS | | (I) Forebrain neural progenitor cells SZ ¹⁶¹ | | | | | 4 |
| | | DE/2 | | | | | | | | | |
| | | 38% | | | | | | | | | |
| **GNG7** G Protein Subunit Gamma 7 | 1566 643_a _at | | 10 | 6.81E-02/2 Stepwi se | | | | | (D) NAC Alcohol ²⁷¹ | | 2 |
| | | (D) | | | | | | | (D) PFC Hallucinogens ¹⁵⁶ | | |
| | | DE/4 | | | | | | | | | |
| | | 59% | | | | | | | (D) PFC (male) BP/Stress ¹³⁶ | | |
| | | | | | | | | | (D) AMY MDD ¹¹¹ | | |
| **COL27A1** Collagen Type XXVII Alpha 1 Chain | 2252 93_at | (D) | 8 | 7.47E-01/2 Stepwi se | Tourette syndrome ²⁷² | | | | | | 2 |
| | | DE/4 | | | | | | | | | |
| | | 79% | | | | | | | | | |
| **DCAF12** DDB1 And CUL4 Associated Factor 12 | 2247 89_at | (D) | 8 | NS | | | (D) SH-SY5Y cells Cocaine ²⁶⁸ | | | | 2 |
| | | DE/6 | | | | | (D) Blood Universal Suicide¹²⁰ | | | | |
| | | 86% | | | | | | | | | |
| **SHMT1** Serine Hydroxym ethyltrans ferase 1 | 2173 04_at | (D) | 8 | NS | | | (D) Blood Suicide ¹²⁹,¹²⁰ | | | | 2 |
| | | DE/2 | | | | | | | | | |
| | | 43% | | | | | | | | | |
| **(Hs.596713 ) PPP1R14B** Protein Phosphata se 1 Regulator y Inhibitor Subunit 14B | 2261 38_s_ at | (D) | 6 | 6.28E-02 Stepwi se | | | | | (D) parietal cortex SZ²⁷³ | | 2 |
| | | DE/6 | | | | | | | | | |
| | | 90% | | | | | | | | | |
| **CCDC85C** Coiled-Coil Domain Containin g 85C | 2190 18_s_ at | (D) | 6 | NS | | | (D) Male Blood Suicide ¹²⁹ | | | | 2 |
| | | DE/6 | | | | | | | | | |
| | | 94% | | | | | | | | | |
| **CLSPN** Claspin | 2421 50_at | (I) | 6 | NS | | | (I) Blood Suicide ^{119, 129} | | | | 2 |
| | | AP/6 | | | | | | | | | |
| | | 95% | | | | | | | | | |
| **ELAC2 ElaC Ribonucle ase Z 2** | 2017 66_at | (D) | 6 | ***4.11E-0214 Nomin al*** | Autism²⁷⁴ | | | | | | 2 |
| | | D E/4 | | | | | | | | | |
| | | 52% | | | | | | | | | |
| **Hs.55426 2** | 2107 03_at | (I) | 6 | NS | | | (I) Blood Universal Suicide¹²⁰ | | | | 2 |
| | | AP/6 | | | | | | | | | |
| **(Hs.65942 6)PHC3** Polyhome otic Homolog 3 | 2405 99_x_ at | (D) | 6 | NS | | | (D) Blood Female Suicide ¹¹⁹ | | | | 2 |
| | | DE/6 | | | | | | | | | |
| **LY9** Lymphocy te Antigen 9 | 2311 24_x_ at | (I) | 6 | NS | | | (D) Blood Stress²⁷⁵ | | | | 2 |
| | | DE/6 | | | | | | | | | |
| | | 90% | | | | | | | | | |
| **MBNL3** Muscleblin d Like | 2198 14_at | (D) | 6 | NS | | | (D) Blood Hallucination s ¹⁴⁷ | | | | 2 |
| | | DE/6 | | | | | | | | | |
| | | 92% | | | | | | | | | |
| Splicing Regulator 3 | | | | | | | | | | | |
| **RALGAPA2** Ral GTPase Activating Protein Catalytic Alpha Subunit 2 | 2318 26_at | (D) | 6 | NS | BP ⁷⁰ | | | | | | 2 |
| | | DE/6 | | | | | | | | | |
| | | 97% | | | | | | | | | |
| **SEPT7P2** Septin 7 Pseudoge ne 2 | 1569 973_a t | **(I)** | 6 | NS | | | (I) Blood Suicide¹¹⁹ | | | | 2 |
| | | **DE/6** | | | | | | | | | |
| | | **100** % | | | | | | | | | |
| | | (I) | | | | | | | | | |
| | | AP/2 | | | | | | | | | |
| | | 39% | | | | | | | | | |
| **TCF15** Transcripti on Factor 15 (Basic Helix-Loop-Helix) | 2073 06_at | (D) | 6 | NS | | | (D) Blood Suicide ¹²⁹, 120 | | | | 2 |
| | | DE/6 | | | | | | | | | |
| | | 94% | | | | | | | | | |
| **TNFRSF11 B TNF Receptor Superfami ly Member 11b** | 2049 32_at | (D) | 4 | ***2.67E-0214 Nomin al*** | | | | | (D) Hippocampus Stress¹²¹ | | 2 |
| | | DE/2 | | | | | | | (D) PFC Stress ²⁵³ | | |
| | | 37% | | | | | | | | | |
| | | | | | | | | | (D) HC PTSD ¹¹⁰ | | |
| **HLA-DRB1** Major Histocomp atibility Complex, Class II, DR Beta 1 | 2083 06_x_ at | (I) | | NS | | | (I) leukocytes Stress ²⁶² | | | | 2 |
| | | AP/4 | | | | | | | | | |
| | | 52% | | | | | (I) Blood PTSD ²⁷⁶ | | | | |
| **CCDC144 B** Coiled-Coil Domain Containin g 144B (Pseudoge ne) | 1557 366_a t | (D) | 10 | NS | | | | | | | 0 |
| | | D E/4 | | | | | | | | | |
| | | 56% | | | | | | | | | |
| **COL2A1** Collagen Type II Alpha 1 Chain | 2174 04_s_ at | (D) | 7 | NS | | | | | | | 0 |
| | | D E/4 | | | | | | | | | |
| | | 54% | | | | | | | | | |
| **(AF090920) PPFIBP2** PPFIA Binding Protein 2 | 2347 39_at | (I) | 6 | NS | | | | | | | 0 |
| | | AP/6 | | | | | | | | | |
| | | 94% | | | | | | | | | |
| **DENND1B** DENN Domain Containin g 1B | 1557 309_a t | (I) | 6 | NS | | | | | | | 0 |
| | | DE/6 | | | | | | | | | |
| | | 90% | | | | | | | | | |
| **ZNF441** Zinc Finger Protein 441 | 1553 193_a t | (I) | 6 | NS | | | | | | | 0 |
| | | AP/6 | | | | | | | | | |
| | | 95% | | | | | | | | | |
| | | (I) | | | | | | | | | |
| | | DE/2 | | | | | | | | | |
| | | 35% | | | | | | | | | |
| **TOP3A** Topoisom erase (DNA) III Alpha | 2143 00_s_ at | (D) | 4 | NS | | | | | | | 0 |
| | | D E/4 | | | | | | | | | |
| | | 51% | | | | | | | | | |
| **ZNF429** Zinc Finger Protein 429 | 1561 270_a t | (D) | 2 | NS | | | | | | | 0 |
| | | DE/2 | | | | | | | | | |
| | | 37% | | | | | | | | | |

| | | | | | | | | | | | |
|---|---|---|---|---|---|---|---|---|---|---|---|
| In the same direction of expression. (I)- increased in expression in Pain, (D)- decreased in expression. DE- differential expression, AP-Absent/Present. | | | | | | | | | | | |

**Table 6. Biological Pathway Analysis:**

| | **DAVID GO Functional Annotation Biological Processes** | | | | | **KEGG Pathways** | | | | **Ingenuity Pathways (Fold change)** | | |
|---|---|---|---|---|---|---|---|---|---|---|---|---|
| A. | # | **Term** | **Count** | **%** | **P-Value** | **Term** | **Cou nt** | **%** | **P-Value** | **Top Canonical Pathways** | **P-Value** | **Overlap** |
| **60 Pain Genes (n= 60 Genes,65 probesets)** | 1 | regulation of homeostatic process | 11 | 18.6 | 1.10E-06 | Focal adhesion | 7 | 11.9 | 7.20E-05 | Hereditary Breast Cancer Signaling | 3.36E-05 | 3.5 % 5/144 |
| | 2 | epithelial cell proliferation | 8 | 13.6 | 9.60E-05 | PI3K-Akt signaling pathway | 8 | 13.6 | 1.60E-04 | Ovarian Cancer Signaling | 3.36E-05 | 3.5 % 5/144 |
| | 3 | T cell receptor signaling pathway | 6 | 10.2 | 1.70E-04 | Non-small cell lung cancer | 4 | 6.8 | 1.00E-03 | Non-Small Cell Lung Cancer Signaling | 4.53E-05 | 5.2 % 4/77 |
| | 4 | aging | 7 | 11.9 | 2.30E-04 | Pancreatic cancer | 4 | 6.8 | 1.60E-03 | Glioblastoma Multiform Signaling | 5.89E-05 | 3.1 % 5/162 |
| | 5 | negative regulation of multicellular organismal process | 12 | 20.3 | 2.50E-04 | Glioma | 4 | 6.8 | 1.60E-03 | HER-2 Signaling in Breast Cancer | 7.65E-05 | 4.5 % 4/88 |

| B. | David | | | | | | | | | Ingenuity Pathways Disease | | |
|---|---|---|---|---|---|---|---|---|---|---|---|---|
| | # | | | Term | Count | | % | | P-Value | Diseases and Disorders | P-Value | # Molecules |
| 60 Pain Genes (n=60 Genes, 65 probesets) | 1 | | | Mood disorders | 5 | | 8.5 | | 2.00 E-05 | Neurological Disease | 2.5 E-05 - 3.26 E-08 | 30 |
| | 2 | | | Head and Neck Cancer | 6 | | 10.2 | | 2.10 E-05 | Cancer | 2.50 E-03 - 9.87 E-08 | 54 |
| | 3 | | | Arthritis, Rheumatoid/Rheumatoid Arthritis | 7 | | 11.9 | | 4.40 E-05 | Organismal Injury and Abnormalities | 2.56 E-03 - 9.87 E-08 | 55 |
| | 4 | | | Autism | 9 | | 15.3 | | 4.40 E-05 | Reproductive System Disease | 1.86 E-03 - 1.79 E-07 | 37 |
| | 5 | | | Glomerulonephritis, IGA | 6 | | 10.2 | | 6.30 E-05 | Renal and Urological Disease | 1.44 E-03 - 1.11 E-06 | 16 |

**Table 7. Pharmacogenomics. Top list biomarkers in datasets that are targets of existing drugs and are modulated by them in opposite direction.**

| **Gene Symbol/Gen e Name Name** | **Probeset** | **Discovery (Change) Method/Sc ore** | **Prioritization Total CFG Score For Pain** | **Validation Anova p-value** | **Pain Medications** | **Omega-3** | **Antidepressants** | **Mood Stabilizer s** | **Antipsyc hotics** | **Others** |
|---|---|---|---|---|---|---|---|---|---|---|
| **CNTN1** Contactin 1 | 1554784 _at | (D) | 10 | NS | | | | | (I) VT Clozapin e 156 | |
| | | D E/4 | | | | | | | | |
| | | 52% | | | | | | | | |
| **GNG7** G Protein Subunit Gamma 7 | 1566643 _a_at | (D) | 10 | 6.81E-02/2 Stepwise | | (I)Brain Omega-3 fatty acids²⁷⁷ | | | | |
| | | D E/4 | | | | | | | | |
| | | 59% | | | | (I)AMY(f emales) Omega-3 fatty²⁷⁸ | | | | |
| **ASTN2** Astrotactin 2 | 1554816 _at | (I) | 8 | 1.71E-01 Stepwise | | | | | Antipsyc hotics ²⁷⁹ | |
| | | DE/6 | | | | | | | | |
| | | 83% | | | | | | | | |
| **CDK6** Cyclin Dependent Kinase 6 | 224851_ at | (I) | 8 | NS | | | | | | palbociclib, ribociclib, abemaciclib, letrozole/pal bociclib, FLX925, fulvestrant/ palbociclib, trilaciclib, G1T38, letrozole/rib ociclib, abemaciclib/ fulvestrant, alvocidib |
| | | D E/4 | | | | | | | | |
| | | 56% | | | | | | | | |
| | | (I) | | | | | | | | |
| | | AP/2 | | | | | | | | |
| | | 42% | | | | | | | | |
| **CDK6** Cyclin Dependent Kinase 6 | 224847_ at | (I) | 8 | NS | | | | | | |
| | | D E/4 | | | | | | | | |
| | | 63% | | | | | | | | |
| **COL27A1** Collagen Type XXVII Alpha 1 Chain | 225293_ at | (D) | 8 | 7.47E-01/2 Stepwise | | | | (I) AMY Lithium 280 | | |
| | | D E/4 | | | | | | | | |
| | | 79% | | | | | | | | |
| **COMT** Catechol-O-Methyltransfe rase | 213981_ at; 216204_ at | (D) | 8 | NS | Morphine ⁴¹ | | | Mood Stabilizer s²⁸¹ | (I) VT Clozapin e ¹⁵⁶ | |
| | | D E/4 | | | Thermal ²¹⁸ | | | | | |
| | | 54% | | | | | | | | |
| **DCAF12** DDB1 And CUL4 Associated Factor 12 | 224789_ at | (D) | 8 | NS | | (I) Lymphoc ytes (females) | | | (I) Lymphoc ytes | |
| | | DE/6 | | | | | | | | |
| | | 86% | | | | Omega-3 fatty acids²⁷⁸ | | | Clozapin e ¹⁵⁶ | |
| **FAh1134B** Family With Sequence Similarity 134 Member B | 218510_ x_at | (I) | 8 | NS | | (D) Lymphoc ytes(females) | | | | |
| | | D E/4 | | | | | | | | |
| | | 51%; (I) | | | | Omega-3 fatty acids²⁷⁸ | | | | |
| | | AP/2 | | | | | | | | |
| | | 34% | | | | | | | | |
| **GBP 1** Guanylate Binding Protein 1 | 231578_ at | (I) | 8 | 3.26E-01/2 Stepwise | | (D) Blood Omega-3 fatty acids ²⁷⁸ | | | | |
| | | DE/2 | | | | | | | | |
| | | 37% | | | | | | | | |
| **HLA-DQB1** Major Histocompati bility Complex, Class II, DQ Beta 1 | 210747_ at | (D) | 8 | NS | | | | | (I)Blood Benzodia zepines ²⁸² | |
| | | DE/2 | | | | | | | | |
| | | 44% | | | | | | | | |
| **HLA-DQB1** Major Histocompati bility Complex, Class II, DQ Beta 1 | 211654_ x_at | (I) | 8 | NS | | | | | (D)PFC Antipsyc hotics ²⁸³ | |
| | | DE/2 | | | | | | | | |
| | | 40% | | | | | | | | |
| **HLA-DQB1** Major Histocompati bility Complex, Class II, DQ Beta 1 | 211656_ x_at; 212998_ x_at | (I) | 8 | NS | | | | | (D) PFC Antipsyc hotics ²⁸³ | |
| | | D E/4 | | | | | | | | |
| | | 59% | | | | | | | | |
| **HLA-DRB1** Major Histocompati bility Complex, Class II, DR Beta 1 | 208306_ x_at | (I) | 8 | NS | | | | | (D)PFC Antipsyc hotics ²⁸³ | apolizumab |
| | | AP/4 | | | | | | | | |
| | | 52% | | | | | | | | |
| **HTR2A** 5-Hydroxytrypt amine Receptor 2A | 211616_ s_at | (D) | 8 | NS | | | | | | Hallucinoge ns |
| | | D E/4 | | | | | | | | |
| | | 52% | | | | | | | | |
| **NF1** Neurofibromi n 1 | 212676_ at | (I) | 8 | NS | | | (D) cerebral cortex Fluoxetine SSRI ²⁸⁴ | | | |
| | | D E/4 | | | | | | | | |
| | | 59% | | | | | | | | |
| **SHMT1** Serine Hydroxymeth yltransferase 1 | 217304_ at | (D) | 8 | NS | | | | | (I)VT Clozapin e ¹⁵⁶ | |
| | | DE/2 | | | | | | | | |
| | | 43% | | | | | | | | |
| **TOP3A** Topoisomeras e (DNA) III Alpha | 214300_ s_at | (D) | 8 | NS | | (I)Brain Omega-3 fatty acids²⁷⁷ | | | | |
| | | D E/4 | | | | | | | | |
| | | 51% | | | | | | | | |
| **VEGFA** Vascular Endothelial Growth Factor A | 212171_ x_at | (I) | 8 | NS | | | | (D) lymphobl astoid cell cultures Lithium, Valproate ²⁸⁵ | (D) HIP and cerebellu m Olanzapi ne ²⁸⁶ | Anti-cancer mABs |
| | | AP/4 | | | | | | | | |
| | | 65% | | | | | | | | |
| **WNK1** WNK Lysine Deficient Protein Kinase 1 | 1555068 _at | (D) | 8 | NS | | (I) Lymphoc ytes(females) | (I) cingulate cortex SSRI (Fluoxetine)²⁶⁴ | | | |
| | | DE/6 | | | | | | | | |
| | | 92% | | | | Omega-3 fatty acids²⁷⁸ | | | | |
| **CALCA** Calcitonin Related Polypeptide Alpha | 210727_ at | (D) | 7 | NS | | (I) HIP (males) | | (I) Schneide r 2 cells Lithium^{28 7} | | |
| | | D E/4 | | | | Omega-3 fatty acids²⁷⁸ | | | | |
| | | 54% | | | | | | | | |
| **ZYX** Zyxin | 238016_ s_at | (D) | 7 | NS | | | | | (I) Lymphoc ytes Clozapin e ¹⁵⁶ | |
| | | D E/4 | | | | | | | | |
| | | 57% | | | | | | | | |
| **(H05785) LRRC75A** Leucine Rich Repeat Conta ining 75A | 236913_ at | (D) | 6 | NS | | | | | (I) HIP Clozapin e¹⁵⁶ | |
| | | AP/6 | | | | | | | | |
| | | 97% | | | | | | | | |
| **(Hs.596713) PPP1R14B** Protein Phosphatase 1 Regulatory Inhibitor Subunit 14B | 226138_ s_at | (D) | 6 | 6.28E-02 Stepwise | | | | (I) Schneide r 2 (S2) cells, Lithium ²⁸⁷ | | |
| | | DE/6 | | | | | | | | |
| | | 90% | | | | | | | | |
| **(Hs.609761) SFPQ** Splicing Factor Proline And Glutamine Rich | 244331_ at | (D) | 6 | NS | | (I) HIP (males) Mood, Omega-3 fatty acids²⁷⁸ | (I) basal forebrain TCA²⁸⁸ | | (I) PFC Clozapin e¹⁵⁶ | |
| | | DE/6 | | | | | | | | |
| | | 98% | | | | | | | | |
| **DENND1B** DENN Domain Containing 1B | 1557309 _at | (I) | 6 | NS | | (D) Brain Omega-3 fatty acids²⁷⁷ | | | | |
| | | DE/6 | | | | | | | | |
| | | 90%; (I) | | | | | | | | |
| | | AP/2 | | | | | | | | |
| | | 40% | | | | | | | | |
| **GSPT1** G1 To S Phase Transition 1 | 215438_ x_at | (D) | 6 | NS | | | | (I) CP Valproate ²⁸⁹ | | |
| | | DE/6 | | | | | | | | |
| | | 94% | | | | | | | | |
| **HRAS** HRas Proto-Oncogene, GTPase | 212983_ at | (I) | 6 | NS | | | | | | ISIS 2503 |
| | | DE/6 | | | | | | | | |
| | | 97% | | | | | | | | |
| **LY9** Lymphocyte Antigen 9 | 231124_ x_at | (I) | 6 | NS | | (D) Brain Omega-3 fatty acids ²⁷⁷ | | | | |
| | | DE/6 | | | | | | | | |
| | | 90% | | | | | | | | |
| **PIK3CD Phosphatidyli nositol-4,5-Bisphosphate 3-Kinase** | 211230_ s_at | (D) | 6 | ***1.59E-0214 Nominal*** | | | | (I) Lymphob lastoid cells Lithium, | (I) VT Clozapin e¹⁵⁶ | |
| | | DE/6 | | | | | | | | |
| | | 83% | | | | | | | | |
| **Catalytic Subunit Delta** | | | | | | | | Valproate 285 | | |
| **PTN** Pleiotrophin | 211737_ x_at | (D) | 6 | NS | | (I) HIP (males) Omega-3 fatty acids ²⁷⁸ (I) fronto-temporo-parietal cortex Antipsyc hotics(ris peridone) ²⁹⁰ | | | | |
| | | DE/6 | | | | | | | | |
| | | 92% | | | | | | | | |
| **SVEP1 Sushi, Von Willebrand Factor Type A, EGF And Pentraxin Domain Containing 1** | 236927_ at | (I) | 6 | ***2.17E-0214 Nominal*** | | (D)Brain Omega-3 fatty acids²⁷⁷ | | | | |
| | | DE/2 | | | | | | | | |
| | | 49% | | | | | | | | |
| **TSPO** Translocator Protein | 202096_ s_at | (I) | 6 | NS | | | | | | CGS-8216, dexamethas one/olanzap ine, fluoxetine/ol anzapine, estazolam, clorazepate, eszopiclone, temazepam, zolpidem, chlordiazepo xide, lorazepam, olanzapine, triazolam, flumazenil, clonazepam, flurazepam, midazolam, flunitrazepa m. |
| | | DE/2 | | | | | | | | |
| | | 38% | | | | | | | | |
| | | | | | | | | | | alprazolam, zaleplon, SSR180575, PK 11195 |
| **YBX3** Y-Box Binding Protein 3 | 201160_ s_at | (D) | 6 | NS | | | (I) c.elegans mianserin ²⁹¹ | | | |
| | | DE/6 | | | | | | | | |
| | | 94% | | | | | | | | |

## Claims

1. A method for determining intensity of pain, the method comprising: quantitatively measuring an expression level of a panel of blood biomarkers personalized by gender and diagnosis in a sample obtained from a subject; obtaining a reference expression level of the blood biomarkers; and identifying a difference in the expression level of the panel of blood biomarkers in the sample and the reference expression level of the blood biomarkers, wherein the difference in the expression level of the panel of blood biomarkers in the sample obtained from the subject and the reference expression level of the blood biomarkers determines the intensity of pain wherein the panel of biomarkers consists of all the biomarkers from the group of biomarkers consisting of GNG7, CNTN1, LY9, CCDC144B, GBP1, Hs.666804/MFAP3, CASP6, COMT, RAB33A, ZYX, Hs.696420/MTERF1, COL27A1, HRAS, CALCA, Hs.596713/PPP1R14B, ASTN2, ELAC2, HLA-DQB1, PNOC, TCF15, TOP3A, H05785/LRRC75A, CLSPN, COL2A1, Hs.554262, PIK3CD, SVEP1, TNFRSF11B, ZNF91, CDK6, EDN1, AF090920/PPFIBP2, DCAF12, DNAJC18, HLA-DRB1, SEPT7P2, VEGFA, WNK1, AF087971/PBRM1, Hs.659426/PHC3, CCDC85C, GSPT1, LOXL2, MBNL3, PTN, RALGAPA2, YBX3, ZNF441, CCND1, Catechol-O-Methyltransferase, HTR2A, NF1, SHMT1, TSPO, DENND1B, MCRS1, OSBP2, FAM134B, ZNF429 and Hs.677263/SMURF2.

2. The method of claim 1, wherein the expression level of the panel of blood biomarkers in the sample obtained from the subject is increased as compared to the reference expression level of the biomarkers or wherein the expression level of the panel of blood biomarkers in the sample obtained from the subject is decreased as compared to the reference expression level of the biomarkers.

3. A method for predicting a future medical care facility visit for pain, the method comprising: quantitatively measuring an expression level of a panel of blood biomarkers personalized by gender and diagnosis in a sample obtained from a subject; obtaining a reference expression level of the blood biomarkers; identifying a difference in the expression level of the panel of blood biomarkers in the sample and the reference expression level of the blood biomarkers, wherein the difference in the expression level of the panel of blood biomarkers in the sample obtained from the subject and the reference expression level of the blood biomarkers determines the likelihood of future medical facility visits for pain wherein the panel of biomarkers consists of all the biomarkers from the group of biomarkers consisting of GNG7, CNTN1, LY9, CCDC144B, GBP1, Hs.666804/MFAP3, CASP6, COMT, RAB33A, ZYX, Hs.696420/MTERF1, COL27A1, HRAS, CALCA, Hs.596713/PPP1R14B, ASTN2, ELAC2, HLA-DQB1, PNOC, TCF15, TOP3A, H05785/LRRC75A, CLSPN, COL2A1, Hs.554262, PIK3CD, SVEP1, TNFRSF11B, ZNF91, CDK6, EDN1, AF090920/PPFIBP2, DCAF12, DNAJC18, HLA-DRB1, SEPT7P2, VEGFA, WNK1, AF087971/PBRM1, Hs.659426/PHC3, CCDC85C, GSPT1, LOXL2, MBNL3, PTN, RALGAPA2, YBX3, ZNF441, CCND1, Catechol-O-Methyltransferase, HTR2A, NF1, SHMT1, TSPO, DENND1B, MCRS1, OSBP2, FAM134B, ZNF429 and Hs.677263/SMURF2.

4. The method of claim 3, wherein the expression level of the panel of blood biomarkers in the sample obtained from the subject is increased as compared to the reference expression level of the biomarkers or wherein the expression level of the panel of blood biomarkers in the sample obtained from the subject is decreased as compared to the reference expression level of the biomarkers.

## Patentansprüche

1. Verfahren zum Bestimmen der Schmerzintensität, das Verfahren umfassend:
quantitatives Messen einer Expressionsrate eines Panels von Blut-Biomarkern, personalisiert durch Geschlecht und Diagnose, in einer Probe, erhalten von einem Subjekt; Erhalten einer Referenz-Expressionsrate der Blut-Biomarker; und
Identifizieren eines Unterschieds zwischen der Expressionsrate des Panels von Blut-Biomarkern in der Probe und der Referenz-Expressionsrate der Blut-Biomarker, wobei der Unterschied zwischen der Expressionsrate des Panels von Blut-Biomarkern in der Probe, erhalten von dem Subjekt, und der Referenz-Expressionsrate der Blut-Biomarker die Intensität des Schmerzes bestimmt, wobei das Panel von Biomarkern aus allen Biomarkern aus der Gruppe von Biomarkern bestehend aus GNG7, CNTN1, LY9, CCDC144B, GBP1, Hs.666804/MFAP3, CASP6, COMT, RAB33A, ZYX, Hs.696420/MTERF1, COL27A1, HRAS, CALCA, Hs.596713/PPP1R14B, ASTN2, ELAC2, HLA-DQB1, PNOC, TCF15, TOP3A, H05785/LRRC75A, CLSPN, COL2A1, Hs.554262, PIK3CD, SVEP1, TNFRSF11B, ZNF91, CDK6, EDN1, AF090920/PPFIBP2, DCAF12, DNAJC18, HLA-DRB1, SEPT7P2, VEGFA, WNK1, AF087971/PBRM1, Hs.659426/PHC3, CCDC85C, GSPT1, LOXL2, MBNL3, PTN, RALGAPA2, YBX3, ZNF441, CCND1, Catechol-O-Methyltransferase, HTR2A, NF1, SHMT1, TSPO, DENND1B, MCRS1, OSBP2, FAM134B, ZNF429 und Hs.677263/SMURF2 besteht.

2. Verfahren nach Anspruch 1, wobei die Expressionsrate des Panels von Blut-Biomarkern in der Probe, erhalten von dem Subjekt, im Vergleich zu der Referenz-Expressionsrate der Biomarker erhöht ist oder wobei die Expressionsrate des Panels von Blut-Biomarkern in der Probe, erhalten von dem Subjekt, im Vergleich zu der Referenz-Expressionsrate der Biomarker verringert ist.

3. Verfahren zum Vorhersagen eines zukünftigen Besuchs einer medizinischen Einrichtung für Schmerzen, das Verfahren umfassend:
quantitatives Messen einer Expressionsrate eines Panels von Blut-Biomarkern, personalisiert durch Geschlecht und Diagnose,, in einer Probe, erhalten von einem Subjekt,; Erhalten einer Referenz-Expressionsrate der Blut-Biomarker;
Identifizieren eines Unterschieds zwischen der Expressionsrate des Panels von Blut-Biomarkern in der Probe und der Referenz-Expressionsrate der Blut-Biomarker, wobei der Unterschied zwischen der Expressionsrate des Panels von Blut-Biomarkern in der Probe, erhalten von dem Subjekt, und der Referenz-Expressionsrate der Blut-Biomarker die Wahrscheinlichkeit zukünftiger Besuche einer medizinischen Einrichtung für Schmerzen bestimmt, wobei das Panel von Biomarkern aus allen Biomarkern aus der Gruppe von Biomarkern bestehend aus GNG7, CNTN1, LY9, CCDC144B, GBP1, Hs.666804/MFAP3, CASP6, COMT, RAB33A, ZYX, Hs.696420/MTERF1, COL27A1, HRAS, CALCA, Hs.596713/PPP1R14B, ASTN2, ELAC2, HLA-DQB1, PNOC, TCF15, TOP3A, H05785/LRRC75A, CLSPN, COL2A1, Hs.554262, PIK3CD, SVEP1, TNFRSF11B, ZNF91, CDK6, EDN1, AF090920/PPFIBP2, DCAF12, DNAJC18, HLA-DRB1, SEPT7P2, VEGFA, WNK1, AF087971/PBRM1, Hs.659426/PHC3, CCDC85C, GSPT1, LOXL2, MBNL3, PTN, RALGAPA2, YBX3, ZNF441, CCND1, Catechol-O-Methyltransferase, HTR2A, NF1, SHMT1, TSPO, DENND1B, MCRS1, OSBP2, FAM134B, ZNF429 und Hs.677263/SMURF2 besteht.

4. Verfahren nach Anspruch 3, wobei die Expressionsrate des Panels von Blut-Biomarkern in der Probe, erhalten von dem Subjekt, im Vergleich zu der Referenz-Expressionsrate der Biomarker erhöht ist oder wobei die Expressionsrate des Panels von Blut-Biomarkern in der Probe, erhalten von dem Subjekt, im Vergleich zu der Referenz-Expressionsrate der Biomarker verringert ist.

## Revendications

1. Procédé pour déterminer une intensité de douleur, le procédé comprenant les étapes consistant à : mesurer quantitativement un niveau d'expression d'un panel de biomarqueurs sanguins personnalisés par sexe et diagnostic dans un échantillon obtenu auprès d'un sujet ; obtenir un niveau d'expression de référence des biomarqueurs sanguins ; et identifier une différence dans le niveau d'expression du panel de biomarqueurs sanguins dans l'échantillon et le niveau d'expression de référence des biomarqueurs sanguins, dans lequel la différence dans le niveau d'expression du panel de biomarqueurs sanguins dans l'échantillon obtenu auprès du sujet et le niveau d'expression de référence des biomarqueurs sanguins détermine l'intensité de la douleur, dans lequel le panel de biomarqueurs est constitué de tous les biomarqueurs du groupe de biomarqueurs constitué de GNG7, CNTN1, LY9, CCDC144B, GBP1, Hs.666804/MFAP3, CASP6, COMT, RAB33A, ZYX, Hs.696420/MTERF1, COL27A1, HRAS, CALCA, Hs.596713/PPP1R14B, ASTN2, ELAC2, HLA-DQB1, PNOC, TCF15, TOP3A, H05785/LRRC75A, CLSPN, COL2A1, Hs.554262, PIK3CD, SVEP1, TNFRSF11B, ZNF91, CDK6, EDN1, AF090920/PPFIBP2, DCAF12, DNAJC18, HLA-DRB1, SEPT7P2, VEGFA, WNK1, AF087971/PBRM1, Hs.659426/PHC3, CCDC85C, GSPT1, LOXL2, MBNL3, PTN, RALGAPA2, YBX3, ZNF441, CCND1, Catéchol-O-Méthyltransférase, HTR2A, NF1, SHMT1, TSPO, DENND1B, MCRS1, OSBP2, FAM134B, ZNF429 et Hs.677263/SMURF2.

2. Procédé selon la revendication 1, dans lequel le niveau d'expression du panel de biomarqueurs sanguins dans l'échantillon obtenu auprès du sujet est augmenté par rapport au niveau d'expression de référence des biomarqueurs, ou dans lequel le niveau d'expression du panel de biomarqueurs sanguins dans l'échantillon obtenu auprès du sujet est diminué par rapport au niveau d'expression de référence des biomarqueurs.

3. Procédé pour prédire une future visite d'établissement de soins médicaux contre la douleur, le procédé comprenant les étapes consistant à : mesurer quantitativement un niveau d'expression d'un panel de biomarqueurs sanguins personnalisés par sexe et diagnostic dans un échantillon obtenu auprès d'un sujet ; obtenir un niveau d'expression de référence des biomarqueurs sanguins ; identifier une différence dans le niveau d'expression du panel de biomarqueurs sanguins dans l'échantillon et le niveau d'expression de référence des biomarqueurs sanguins, dans lequel la différence dans le niveau d'expression du panel de biomarqueurs sanguins dans l'échantillon obtenu auprès du sujet et le niveau d'expression de référence des biomarqueurs sanguins détermine la probabilité de futures visites dans un établissement médical contre la douleur, dans lequel le panel de biomarqueurs se compose de tous les biomarqueurs du groupe de biomarqueurs constitué de GNG7, CNTN1, LY9, CCDC144B GBP1, Hs.666804/MFAP3, CASP6, COMT, RAB33A, ZYX, Hs.696420/MTERF1, COL27A1, HRAS, CALCA, Hs.596713/PPP1R14B, ASTN2, ELAC2, HLA-DQB1, PNOC, TCF15, TOP3A, H05785/LRRC75A, CLSPN, COL2A1, Hs.554262, PIK3CD, SVEP1, TNFRSF11B, ZNF91, CDK6, EDN1, AF090920/PPFIBP2, DCAF12, DNAJC18, HLA-DRB1, SEPT7P2, VEGFA, WNK1, AF087971/PBRM1, Hs.659426/PHC3, CCDC85C, GSPT1, LOXL2, MBNL3, PTN, RALGAPA2, YBX3, ZNF441, CCND1, Catéchol-O-Méthyltransférase, HTR2A, NF1, SHMT1, TSPO, DENND1B, MCRS1, OSBP2, FAM134B, ZNF429 et Hs.677263/SMURF2.

4. Procédé selon la revendication 3, dans lequel le niveau d'expression du panel de biomarqueurs sanguins dans l'échantillon obtenu auprès du sujet est augmenté par rapport au niveau d'expression de référence des biomarqueurs, ou dans lequel le niveau d'expression du panel de biomarqueurs sanguins dans l'échantillon obtenu auprès du sujet est diminué par rapport au niveau d'expression de référence des biomarqueurs.
